# EUROPÄISCHE PATENTSCHRIFT

(11) **EP 1 177 320 B1**
(45) Veröffentlichungstag und Bekanntmachung des Hinweises auf die Patenterteilung: **25.02.2004**
(21) Anmeldenummer: 00931021.0
(22) Anmeldetag: 04.04.2000
(51) Int. Cl.: C12Q 1/70

(54) **VERFAHREN ZUM SPEZIFISCHEN NACHWEIS UND ZUR IDENTIFIZIERUNG RETROVIRALER NUKLEINSÄUREN/RETROVIREN IN EINEM UNTERSUCHUNGSGUT**
METHOD FOR SPECIFICALLY DETECTING AND IDENTIFYING RETROVIRAL NUCLEIC ACIDS/RETROVIRUSES IN AN ITEM TO BE EXAMINED
PROCEDE DE DETECTION SPECIFIQUE ET D'IDENTIFICATION D'ACIDES NUCLEIQUES RETROVIRAUX / DE RETROVIRUS DANS UN ELEMENT A ANALYSER

(30) Priorität: 08.05.1999 DE 19921419
(43) Veröffentlichungstag der Anmeldung: 06.02.2002
(73) Patentinhaber: Ruprecht-Karls-Universität Heidelberg, 69117 Heidelberg (DE)
(72) Erfinder: SEIFARTH, Wolfgang, D-69226 Nussloch (DE); LEIB-MÖSCH, Christine, D-80809 München (DE); BAUST, Corinna, D-69190 Walldorf (DE)
(74) Vertreter: Rudolph, Ulrike, Dr.
(86) Internationale Anmeldenummer: PCT/DE2000/001071
(87) Internationale Veröffentlichungsnummer: WO 2000/068435

(56) Entgegenhaltungen:
- EP-A- 0 229 701
- EP-A- 0 789 081
- WO-A-93/25707
- WO-A-95/02704
- US-A- 5 478 724
- MACK D H ET AL: "A SENSITIVE METHOD FOR THE IDENTIFICATION OF UNCHARACTERIZED VIRUSES RELATED TO KNOWN VIRUS GROUP HEPADNAVIRUS MODEL SYSTEM" PROCEEDINGS OF THE NATIONAL ACADEMY OF SCIENCES OF THE UNITED STATES, Bd. 85, Nr. 18, 1988, Seiten 6977-6981, XP002148107 1988 ISSN: 0027-8424
- SEIFARTH WOLFGANG ET AL: "Retrovirus-like particles released from the human breast cancer cell line T47-D display type B- and C-related endogenous retroviral sequences." JOURNAL OF VIROLOGY, Bd. 69, Nr. 10, 1995, Seiten 6408-6416, XP002148206 ISSN: 0022-538X
- DONEHOWER L A ET AL: "THE USE OF PRIMERS FROM HIGHLY CONSERVED POL REGIONS TO IDENTIFY UNCHARACTERIZED RETROVIRUSES BY THE POLYMERASE CHAIN REACTION" JOURNAL OF VIROLOGICAL METHODS, Bd. 28, Nr. 1, 1990, Seiten 33-46, XP000946504 ISSN: 0166-0934
- ROSE TIMOTHY M ET AL: "Consensus-degenerate hybrid oligonucleotide primers for amplification of distantly related sequences." NUCLEIC ACIDS RESEARCH, Bd. 26, Nr. 7, 1. April 1998 (1998-04-01), Seiten 1628-1635, XP002148108 ISSN: 0305-1048
- WICHMAN H A ET AL: "IN SEARCH OF RETROTRANSPOSONS EXPLORING THE POTENTIAL OF THE PCR" BIOTECHNIQUES, Bd. 13, Nr. 2, 1992, Seiten 258-263, 265, XP002148109 ISSN: 0736-6205
- INNIS ET AL.: "PCR PROTOCOLS" 1990 , ACADEMIC PRESS , US,SAN DIEGO XP002148111 "DETECTION AND TYPING OF GENITAL HUMAN PAPILLOMAVIRUS", TING ET AL. Seite 356 -Seite 367
- INNIS ET AL.: "PCR PROTOCOLS" 1990 , ACADEMIC PRESS , US,SAN DIEGO XP002148112 "DEGENERATED PRIMERS FOR DNA AMPLIFICATION" ,COMPTON Seite 39 -Seite 45
- SEIFARTH WOLFGANG ET AL: "Rapid identification of all known retroviral reverse transcriptase sequences with a novel versatile detection assay." AIDS RESEARCH AND HUMAN RETROVIRUSES, Bd. 16, Nr. 8, 20. Mai 2000 (2000-05-20), Seiten 721-729, XP002148110 ISSN: 0889-2229

## Beschreibung

Die Erfindung betrifft eine aus Vorwärts- und Umkehrprimern bestehende Primermischung (MOP) für die PCR, ein Verfahren zum spezifischen Nachweis und zur Identifizierung retroviraler Nukleinsäuren / Retroviren in einem Untersuchungsgut sowie Diagnosekits zur Durchführung dieses Verfahrens. Sie betrifft außerdem retrovirusspezifische Sonden für die Reverse Dot Blot Hybridisierung.

Exogene und endogene Retroviren (HERV) sind ätiologisches Agenz für eine Vielzahl von tumorigenen Erkrankungen bei Mensch und Tier. In zahlreichen Tiermodellen, aber auch beim Menschen (HTLV-I und II) sind sie an der Entstehung von Tumoren und Leukämien beteiligt. Andere hingegen verursachen Immundefizienzerkrankungen (HIV). Gegenwärtige Untersuchungen lassen den Schluß zu, daß Retroviren auch als Auslöser von Autoimmunerkrankungen (Kalden und Herrmann, 1993) und neuronalen degenerativen Erkrankungen wie multipler Sklerose (Tuke et al. 1997) eine Rolle spielen können. Intensive Forschung auf dem Gebiet der endogenen und exogenen Retroviren führte bislang zur Entdeckung laufend neuer retroviraler Sequenzen im menschlichen Erbgut, deren Expression möglicherweise mit bestimmten Erkrankungen assoziiert sein könnte. So ist die Expression von Gag-Proteinen der HERV-K Familie mit fast allen Formen testikulärer und ovarieller Keimzelltumoren assoziiert (Sauter et al. 1995). In menschlichen Seren wurden Antikörper gegen HERV-K Env Protein nachgewiesen (Vogetseder et al. 1993). Das *env* Gen von HERV-K-IDDM, das aus Patienten mit Diabetes Typ-1 isoliert wurde, kodiert möglicherweise für ein endogenes Superantigen (Conrad et al. 1997).

Zur Korrelation bestimmter Erkrankungen mit der Aktivität bestimmter endogener oder exogener Retroviren sind statistisch abgesicherte Studien mit großen Patientenkollektiven notwendig. Der hierfür erforderliche Zeit- und Kostenaufwand mit den im Stand der Technik bekannten Nachweisverfahren ist immens.

Der zunehmende Einsatz retroviraler Vektorsysteme in der humanen Gentherapie wirft Fragen hinsichtlich der Sicherheit vor unerwünschten Nebenwirkungen (Genomveränderungen in den Zielzellen, Übertragung unerwünschter Viren) auf So werden in Verpackungszellinien zu einem gewissen Prozentsatz auch unerwünschte Genabschnitte endogener oder fremder Retroviren in die therapeutisch zu applizierenden retroviralen Partikel mitverpackt (Co-packaging, Sherwin et al. 1987, Scolnick et al. 1979, Takeuchi et al. 1992). So konnten Transkripte bestimmter endogener Retroviren, wie sie in verwandter Form auch im Genom von Verpackungszellinien vorhanden sind, in Retrovirus-ähnlichen Partikeln (Pseudotypen) der Brustkrebszellinie T47D nachgewiesen werden (Seifarth et al. 1995, 1998). Die Verpackung solcher unerwünschten retroviralen Sequenzen kann zur Rekombination und zur Entstehung neuer Retroviren mit veränderten, möglicherweise pathogenen Eigenschaften, fuhren. Die Neuintegration solcher rekombinanten Retroviren im Genom der Zielzellen kann zu Insertionsmutagenese und dadurch zur Inaktivierung wichtiger Gene des Zellzyklus und möglicherweise zur Tumorgenese führen.

Aus diesem Grund ist es notwendig, mit einem empfindlichen Testsystem eine Qualitätskontrolle der gentherapeutisch einzusetzenden Genvektorpräparationen durchzuführen. Damit könnte vermieden werden, daß unerwünschte retrovirale Sequenzen transfundiert werden. Bei positivem Nachweis könnte eine Vektorpräparation vor der Gabe an den Patienten noch einer entsprechenden Reinigung (Purging) unterzogen werden. Die im Stand der Technik bislang bekannten Verfahren sind für einen solchen Einsatz nicht geeignet.

Eine gegenwärtig umstrittene Frage ist der Einsatz von Tierorganen für die Transplantation am Menschen (Xenotransplantation). So werden aus Ermangelung geeigneter Spender dem Menschen vermehrt Herzklappen von Schweinen transplantiert. Auch die Übertragung von Herz-, Leber- und Nieren-Transplantaten ist geplant. Jüngste Untersuchungen ergaben aber, daß es im Rahmen der Transplantation und der damit verbundenen medikamentösen Immunsuppression beim Empfänger zur Aktivierung endogener oder bislang supprimierter exogener Retroviren im Spenderorgan kommen kann. Wie experimentell bereits nachgewiesen wurde, sind diese Retroviren tierischen Ursprungs pathogen für bestimmte menschliche Zelltypen (Xenotropismus) und könnten somit zu einer ernsthaften systemischen Erkrankung des Organempfängers führen. Im Falle der Bildung pathogener infektiöser Viruspartikel ist auch eine Übertragung auf unbeteiligte Dritte (Epidemie) nicht auszuschließen. Nicht zuletzt könnten Rekombinationen von Retroviren tierischen Ursprungs mit endogenen humanen Retroviren zu neuen pathogenen Virusrekombinanten mit völlig neuen Wirtstropismen führen.
Es besteht daher der Bedarf an schnellen, zuverlässigen und zugleich preisgünstigen Detektionssystemen, mit dem Transplantat-Träger regelmäßig auf eine Infektion mit Retroviren tierischen Ursprungs getestet werden können.

Für den Nachweis viraler Infektionen stehen im Stand der Technik bislang eine Reihe von Methoden des direkten und indirekten Virus-Nachweises zur Verfügung. Zum direkten Nachweis von Viruspartikeln, Produkten der viralen Replikation (virale Antigene) oder einer gegen das Virus gerichtete Immunantwort (antivirale Antikörper), gehören Elektronenmikroskopie (EM), Färbung viraler Proteine mit fluoreszierenden Antikörpern, "enzyme-linked immunosorbent assay" (ELISA) und Radioimmunassays (RIA). Zum direkten Nachweis des Virus und seiner Nukleinsäuren werden auch zunehmend molekularbiologische Methoden, wie Nukleinsäurehybridisierungen mit Virus-spezifischen Gensonden (Dot-Blot, Southern-Blot, Northem-Blot) sowie die Polymerase-Kettenreaktion (PCR) mit Virus-spezifischen Primem durchgeführt.

Mit den indirekten Methoden werden in der Regel nicht die Viren selbst, sondern ihre Folgeerscheinungen, d. h. die durch eine Virusvermehrung (Replikation) induzierten Veränderungen (zytopathische Effekte) in Zellen nachgewiesen. Dies muß meist in einem auf das nachzuweisende Virus zugeschnittenen *in vitro* Zellkultursystem durchgeführt werden. Dafür werden lebende Zellen, in denen das nachzuweisende Virus replizieren kann, benötigt. Je nach Virustyp sind Zellkulturen, Organkulturen, befruchtete Hühnereier oder sogar Labortiere für den Nachweis erforderlich. Das Erscheinungsbild des zytopathischen Effekts (Zell-Lyse, fokales oder diffuses Zellwachstum, Synzytienbildung, Abrundung) und das Wirtsspektrum des Virus wird als Indiz für die Identifizierung des Virusisolates herangezogen. Oft aber ist die genaue Identifizierung nur in Kombination mit serologischen oder molekularbiologischen Methoden (PCR) möglich.

Die relativ geringe Sensitivität einiger direkter Nachweismethoden (EM, Antikörperfärbung) macht es notwendig, daß das Untersuchungsmaterial für einen erfolgreichen Nachweis eine bestimmte Menge an Virus enthalten muß oder durch geeignete Methoden (Ultrazentrifugation) angereichert werden muß. Ist dies nicht praktizierbar, muß das Virus vorher in einem speziellen *in vitro* Zellkultursystem angezüchtet werden. Da viele Viren spezielle Wirtszelltropismen besitzen, ist für jedes zu testende Virus ein spezielles Testsystem notwendig. Dies resultiert in hohen Laborkosten, deren Auswertung zum Teil sehr zeitintensiv ist und sehr viel Erfahrung erfordert.
Serologische Methoden (ELISA, RIA) sind in der Regel hochsensitiv und haben sich zum gängigen Goldstandard in der Virusdiagnostik entwickelt. Der Nachteil aller serologischer Methoden ist aber, daß für jeden zu testenden Virus ein spezifischer Antikörper notwendig ist. In einem Testdurchlauf kann die zu untersuchende Probe somit nur auf ein putatives Virus getestet werden. Die Untersuchungen von ganzen Expressionsmustern ist mit diesen Methoden nur unter großem zeitlichen und finanziellen Aufwand möglich.
Entwicklungen auf dem Gebiet der Molekularbiologie haben zur Entwicklung neuer Nachweismethoden (Hybridisierungen, PCR) geführt, die eine ähnliche Empfindlichkeit wie serologische Antigen-Nachweismethoden besitzen. Auch hier steht und fällt der Nachweiserfolg mit der Verfügbarkeit Virus-spezifischer Gensonden (Hybridisierung) oder Oligonukleotide (PCR). Da der Einsatz mehrerer Sonden oder PCR-Primer aufgrund unspezifischer Wechselwirkungen in einem Reaktionsansatz limitiert ist, müssen zum Nachweis komplexer Expressionmuster ebenfalls viele Experimente parallel durchgeführt werden.

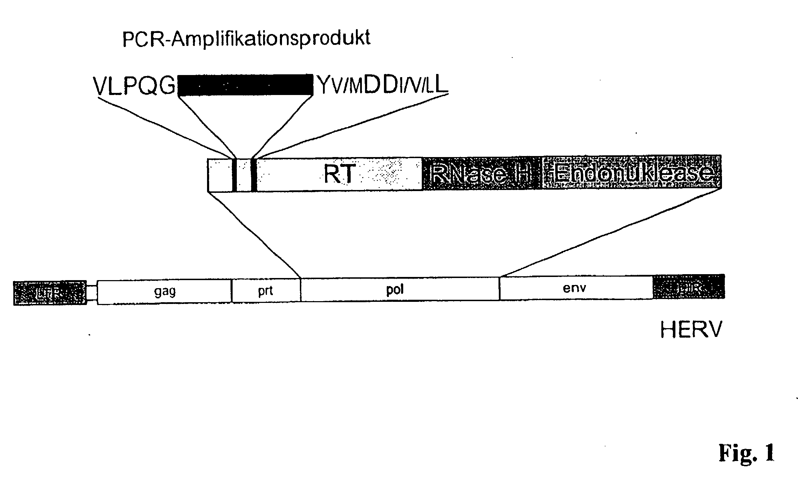

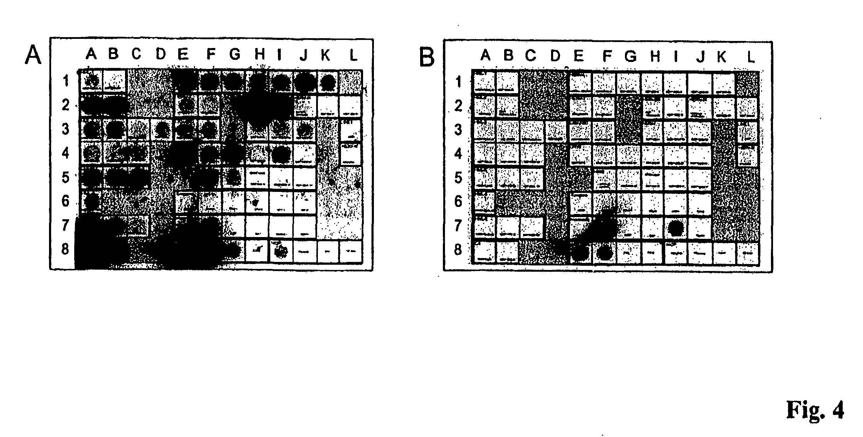

Mack et al. (PNAS 1988, 85 [18] 6977-6981) beschreiben degenerierte PCR-Primer, die angeblich den Nachweis von Hepatitis-Viren einschließlich bislang unbekannter Hepatitis-Viren und nahe verwandter Onkoviren ermöglichen. Diese Primer sind von vier charakterisierten Hepatitis-DNA-Viren abgeleitet. Die Funktionsüberprüfung der Primermischungen erfolgte jeweils an 1 µg menschlicher genomischer DNA, die mit je 300.000 Plasmidkopien des entsprechenden nachzuweisenden Hepatitis-DNA-Virus gemischt war, und nur in einfachen Amplifikationsreaktionen, in denen jeweils nur eine Zielsequenz amplifiziert werden mußte. Da in den beschriebenen PCR-Funktionstestansätzen jedoch 1 µg menschlicher genomischer DNA eingesetzt wurde, hätten bei effizienter Amplifikation von Retroviren eine Vielzahl endogener Retroviren mit amplifiziert werden müssen, da ca. 8% des menschlichen Genoms aus solchen Sequenzen besteht. Diese Amplifikationsprodukte hätten unbedingt als alles überlagernder Hintergrund sichtbar sein müssen. Die Abbildung 4 belegt aber eindeutig, dass dem nicht so ist, sondern praktisch nur die Zielsequenzen amplifiziert worden sind. Der Nachweis, daß die Primer in der Lage sind, aus einem komplexen Gemisch von Zielsequenzen alle Onkoviren und Hepatitisviren mit gleicher Effizienz zu amplifizieren und nachzuweisen, wird von Mack et al. infolgedessen nicht erbracht.
Donehower et al. (Virological Methods 1990, 28 [1] 33-46) beschreiben eine Mischung degenerierter PCR-Primer, die von 8 charakterisierten Retroviren aus Mensch und Tier (Seite 37, Fig 1) abgeleitet wurden. Die Wirksamkeit der Primer wurde nicht mit komplexen Zielsequenzgemischen sondern mit 4 einzelnen Virusklonen/Virusisolaten getestet. Tests mit genomische DNA vom Menschen zwecks Klärung der Frage, ob humane endogene Retroviren (HERV) auch amplifiziert werden können, wurden nicht durchgeführt Die Nachweisempfindlichkeit für Retroviren mittels dieser Primer wird von Donehower et al. mit 5x10⁵ Virusgenome angegeben, was für eine PCR als recht gering einzustufen ist. Bisher gibt es keine Testergebnisse, die zeigen, daß diese Primer in der Lage sind, das gesamte Spektrum endogener Retroviren im menschlichen Genom zu amplifizieren. Vielmehr weisen eigene Untersuchungen darauf hin, daß mit diesen Primern aus komplexen humanen cDNA-Gemischen und genomischer DNA immer nur bevorzugt Retroviren vom Typ C amplifiziert werden. Insbesondere B-Typ-verwandte HERV-Familien waren in den analysierten Amplifikaten stark unterrepräsentiert. Einige Virustypen, die im Northemblot deutlich erkennbar waren, konnten mit diesen Primern überhaupt nicht aus mRNA amplifiziert werden.
In der Publikation von Seifarth et al. (J Virology 1995, 69 [10] 6408-6416) wird die Isolierung und Charakterisierung unbekannter Retrovirus-Genome aus retroviralen Partikeln (T47D-Partikel) aus Kulturüberständen der humanen Mammakarzinomzellinie T47D beschrieben. Dabei wurde eine Primermischung verwendet, die im wesentlichen aus den von Donehower et al. (a.a.O.) beschriebenen Primern zusammengesetzt war. Mit diesen Primern konnte eine Teil der in den T47D-Partikeln verpackten Sequenzen amplifiziert werden, nicht jedoch das gesamte Spektrum an humanen endogenen Retrovirus (HERV) -Sequenzen, die in T47D-Zellen exprimiert werden. Die amplifizierten Sequenzen stellen kein komplexes Gemisch von Zielsequenzen dar, sondern repräsentieren nur einen sehr geringen Teil der im Genom der Zellen vorhandenen und aktiv exprimierten HERV-Sequenzen.

Angesichts der vorstehend geschilderten Umstände war es Aufgabe der vorliegenden Erfindung, eine Primermischung für die PCR sowie ein leistungsfähiges, zuverlässiges und zugleich schnelles Verfahren zum multiplen Nachweis endogener und exogener Retroviren menschlichen und tierischen Ursprungs bereitzustellen.

Diese Aufgabe wird mit einer Primermischung der eingangs genannten Art gelöst, die dadurch gekennzeichnet ist, daß Vorwärtsprimer und Umkehrprimer degenerierte Oligonukleotide sind, daß entweder ("MOP-ABD") die Vorwärtsprimer eine Nukleotidsequenz aufweisen, die aus der Nukleotidsequenzgemäß SEQ ID NO. 1 mit 3456 Degenerationen und einem "Kopf" am 5'-Ende besteht, und die Umkehrprimer eine Nukleotidsequenz aufweisen, die aus der Nukleotidsequenz gemäß SEQ ID NO. 2 mit 27648 Degenerationen und einem "Kopf" am 5'-Ende besteht, oder daß ("MOP-C") die Vorwärtsprimer eine Nukleotidsequenz aufweisen, die aus der Nukleotidsequenz gemäß SEQ ID NO. 3 mit 3072 Degenerationen und einem "Kopf" am 5'-Ende besteht, und die Umkehrprimer eine Nukleotidsequenz aufweisen, die aus der Nukleotidsequenz gemäß SEQ ID NO. 4 mit 8192 Degenerationen und einem "Kopf" am 5'-Ende besteht, und daß "Kopf" für eine Nukleotidsequenz steht, die eine Schnittstelle für ein Restriktionsenzym und eine Klammer-Sequenz, die der Stabilisierung der Schnittstellen sequenz dient, am 5'-Ende dieser Schnittstelle umfaßt, wobei die Länge dieser Nukleotidsequenz die halbe Länge der kompletten Nukleotidsequenz des Vorwärts- oder Umkehrprimers nicht überschreitet.

Diese Aufgabe wird ferner mit einem Verfahren der eingangs genannten Art gelöst, das dadurch gekennzeichnet ist, daß es die folgenden Maßnahmen umfaßt:
- Isolierung der Nukleinsäuren, nämlich DNS- und/oder RNS, aus dem Untersuchungsgut,
- Durchführung einer PCR mit den isolierten DNS oder einer RT-PCR mit den isolierten RNS unter Verwendung von einer oder beiden der vorstehend genannten erfindungsgemäßen Primermischungen MOP-ABD und MOP-C,
- Aufreinigung der erhaltenen (RT-) PCR-Amplifikate und Einsatz derselben in einem Reverse-Dot-Blot-Hybridisierung (RDBH)-Verfahren unter Verwendung immobilisierter Reverse-Dot-Blot-Hybridisierungs (RDBH)-Sonden, wobei diese Sonden synthetische Oligonukleotide umfassen, deren Nukleotidsequenz der retroviralen Nukleotidsequenz des retroviruspezifischen Reverse-Transkriptase-Gens der mit dem betreffenden Dot nachzuweisenden Virusart oder einem Abschnitt einer solchen retroviralen Nukleotidsequenz entspricht und keine Überlappung mit den Nukleotidsequenzen der in der PCR oder RT-PCR eingesetzten Vorwärtsprimern (forward primer) und Umkehrprimern (reverse primer) aufweist.
Mit anderen Worten: Bei dem erfindungsgemäßen Verfahren zum Nachweis von retroviralen Nukleinsäuren in einer Probe, werden zuerst alle Nukleinsäuren (RNA und DNA) unter Einsatz gängiger, dem Fachmann bekannten Methoden, aus dem Untersuchungsgut extrahiert. Hierbei wird zwischen DNA und RNA unterschieden. Für den Nachweis von bereits ins Wirtszellgenom integrierten Retroviren (Proviren) ist die Isolation von genomischer DNA ausreichend. Soll die Aktivierbarkeit bislang ruhender Retroviren, die Transkriptionsaktivität endogener Retroviren oder die Identität retroviraler Partikel untersucht werden, muß polyadenylierte Boten-RNA (mRNA) isoliert werden, die frei von genomischer DNA ist. Im Falle der Verwendung von mRNA als Ausgangsmaterial muß diese mRNA *in vitro* mittels Reverser Transkriptase in komplementäre DNA (cDNA) umgeschrieben werden und kann dann als Matrize für die sich anschließende PCR eingesetzt werden. Diese Kombination von reverser Transkription und PCR wird gemeinhin als RT-PCR bezeichnet.
Die isolierten Nukleinsäuren werden anschließend einer Ein-Schritt PCR unter Verwendung der erfindungsgemäßen Primermischungen (MOP-ABD, MOP-C) aus retrovirusspezifischen, degenerierten, zu den hochkonservierten Regionen innerhalb des Reverse Transkriptase Gens (RT-Gens) aller bekannten humanen Retroviren korrespondierenden Oligonukleotiden (MOP, Shih et al. 1989, Donehower et al. 1990), unterworfen. Bei dieser PCR werden sämtliche retrovirusspezifischen 'Reverse Transkriptase' - homologen Sequenzabschnitte, die im Untersuchungsgut enthalten sind, amplifiziert. Als Ergebnis erhält man ein Amplifikatgemisch kurzer retroviraler DNA-Fragmente, das in seiner Zusammensetzung die Häufigkeit aller nachzuweisenden retroviralen Nukleotidequenzen in dem Untersuchungsgut wiederspiegelt. Die Amplifikate werden entweder bereits während der PCR-Reaktion oder im Anschluß daran markiert, vorzugsweise radioaktiv, wahlweise aber ebensogut auch nichtradioaktiv (z.B.mit Biotin oder Digoxigenin). Anschließend werden diese markierten Amplifikate in einem Hybridisierungsverfahren (RDBH-Verfahren) unter Verwendung von Filtermembranen oder Bio-Chips mit aufgebrachten, retrovirusspezifischen Oligonukleotiden als Sonden eingesetzt.
Die aus Klammer- und Schnittstellensequenz bestehende Kopf- bzw. Extensionssequenz der erfindungsgemäßen Primeroligonukleotide, hat zum einen den positiven Effekt, daß sie die Primer-Matrizen-Bindungskinetik günstig beeinflußt, so daß die in dem ersten PCR-Zyklus gebildeten PCR-Produkte in den folgenden Zyklen noch wesentlich effizienter amplifiziert werden. Damit ist der Vorteil verbunden, daß retrovirale Matrizen selbst dann amplifiziert werden bzw. werden können, wenn der exakt dazu passende Primer nicht in der Primermischung vorhanden ist. Die Schnittstelle hat außerdem den Vorteil, daß sie im Bedarfsfall die Durchführung einer Klonierung erleichtert.
Grundsätzlich gilt, daß die Länge der Kopf- bzw. Extensionssequenz die halbe Länge der kompletten Primernukleotidsequenz nicht überschreiten sollte.

Wesentlicher Bestandteil des erfindungsgemäßen Verfahrens sind die für die Reverse Dot Blot Hybridisierung (RDBH) verwendeten RDBH-Sonden, bei denen es sich um bestimmte Mengen synthetisch erzeugter, genau definierter Nukleinsäuresequenzen aus dem Reverse Transkriptase Gen derjenigen bereits charakterisierten Retroviren handelt, gegen die das Untersuchungsgut getestet werden soll. Diese retrovirusspezifischen RDBH-Sonden sind auf definierten Feldern (Dots) der RDBH-Träger aufgebracht, wobei es sich sowohl um althergebrachte Filtermembranen mit Abmessungen von mehreren Zentimetern handelt kann, als auch um sogenannte Bio-Chips mit Abmessungen von wenigen Millimetern ("Micro-Array-Technologie"). Im Fall von Filtermembranen werden die Sonden vorzugsweise kovalent durch UV-Crosslinking (z.B. mit der kommerziell erhältlichen UV-Strahlungsquelle 'Stratalinker™', Stratagene) an diesen Träger gebunden..
Bei den DNA-Chips werden Oligonukleotid-Sonden in situ synthetisiert und mit genau definierten Positionen auf einem festen Träger mit Methoden der Photolitographie (ähnlich einer Gravur) fixiert, (z.B. indem unter Verwendung von Lochmasken bestimmte Bereiche des Chips belichtet werden, um photosensitive chemische Gruppen zu aktivieren). Der Träger, vorzugsweise eine Glas- oder Nylonoberfläche von ca. 1 cm². bildet die Hybridisierungseinheit. Jede Hybridisierungseinheit kann eine sehr hohe Zahl an unterschiedlichen Ologonukleotid-Sonden (bis zu 400.000) enthalten. Dadurch wird die gleichzeitige Analyse von vielen tausend verschiedenen Sequenzen ermöglicht. Für jede Sonde werden alle Sequenz-Alternativen auf dem Chip dargestellt, eine einzige davon muß von dem zu testenden Untersuchungsgut erkannt werden. Die Hybridisierung von Sonde und Zielsequenz (im Untersuchungsgut) wird über die Messung der Amplifikatmarkerintensität nachgewiesen. Die Intensität ist proportional zum Ausmaß der Hybrisisierung zwischen Sonde und Zielsequenz. Jede Zielsequenz wird nach ihrer Hybridisierungsposition auf dem Chip identifiziert. Die Technik der DNA-Chips wurde von FODOR et al. ( Science 251, 767-773, 1991) entwickelt und ist im Stand der Technik bekannt (vgl. V. Oeding et al., 1999, *HYGIENE UND MIKROBIOLOGIE* 1/99, S. 55-57 und G. Ramsay 1998, *NATURE BIOTECHNOLOGIE,* Vol. 16, 1998, S.40-44.).
Die Hybridisierung sollte unter hochstringenten, auf die Länge der Sonden abgestimmten Bedingungen erfolgen, und die hybridisierten Träger (Filtermembranen oder Chips) sollten anschließend unter Bedingungen hoher Stringenz gewaschen werden. Die Identität der nachweisbaren Retroviren kann - im Fall einer radioaktiven Markierung der PCR-Amplifikate - nach Exposition der Filtermembranen auf einem Röntgenfilm anhand der Signalmuster der Autoradiogramme identifiziert werden.

Das erfindungsgemäße Verfahren ermöglicht den multiplen Nachweis und die Identifizierung aller bislang bekannten humanen und/oder tierspezifischen retroviralen Nukleinsäuren/Retroviren in Zellkulturen, Zellkulturüberständen oder Körperproben oder sonstigem Untersuchungsgut biologischen Ursprungs. Voraussetzung ist lediglich, daß bestimmte Genomabschnitte der nachzuweisenden Retroviren, nämlich die konservierten Sequenzbereiche der Reversen Transkriptase, hinsichtlich ihrer DNA-Nukleotidsequenz bekannt sind. Diese Voraussetzung ist erfüllt, da die entsprechenden Nukleotidsequenzen exogener und endogener humaner Retroviren als Genbankdaten allgemein zugänglich sind.

Das erfindungsgemäße Verfahren eröffnet erstmals die Möglichkeit der Bereitstellung eines universellen Retrovirus-Detektionssystems, mit dessen Hilfe in einem einzigen Experiment das gesamte Spektrum (= Expressionsmuster) aller aktiven endogenen und exogenen retroviralen Nukleotidsequenzen im Untersuchungsgut (Körperprobe) erfaßbar ist. Mit diesem Detektionssystem können insbesondere auch statistisch abgesicherte Studien mit beliebig großen Patientenkollektiven durchgeführt werden und daraus gegebenenfalls bestehende Korrelationen zwischen bestimmten Erkrankungen und der Aktivität bestimmter endogener oder exogener Retroviren festgestellt werden. Im Falle einer nachgewiesenen Korrelation eines bestimmten retroviralen Expressionsmusters mit einer bestimmten Erkrankung kann dieses Testsystem auch zur Früherkennung bzw. zur Abschätzung des persönlichen genetischen Risikos für eine solche Erkrankung eingesetzt werden.

Ein weiterer ganz entscheidenderer Vorteil des Verfahrens liegt darin, daß die erfindungsgemäßen PCR-Primer (MOP), d.h. die degenerierten Oligonukleotide der Primermischungen, die für die Amplifikation der zu identifizierenden retrovirusspezifischen RT-Genabschnitte im Untersuchungsgut verwendet werden, in ihrer Sequenz nicht mit den Sequenzen der als Dot Blot Sonden eingesetzten synthetischen virusspezifischen Oligomere überlappen. Die PCR-Primersequenzen tragen nämlich ungefähr zur Hälfte der endgültigen Amplifikatlänge bei. Wären diese Sequenzabschnitte auch in den Dotblot-Sonden enthalten (vgl. Herrmann und Kalden, 1994), würde dies zu erheblichen Einschränkungen in der Aussagekraft des Tests führen, da die Amplifikate dann zu einem gewissen Maße mit allen Dotblot-Sonden auf der Filtermembran hybridisieren würden. Es ist der Vorteil des erfindungsgemäßen Verfahrens, daß dieser unerwünschte Effekt durch die Verwendung synthetischer, präzise definierter, homogener Oligonukleotidpräparationen für Vorwärts- (forward) und Umkehrprimer (reversed primer) einerseits und für die RDBH-Sonden andererseits verhindert wird.

Die Nukleotidsequenzen aller bislang charakterisierten exogenen und endogenen Retroviren (HERV) sind in Genbanken publiziert. Von ihnen können entsprechende Nukleotidsequenzen für die Synthese virusspezifischer Oligomere als Dot Blot-Sonden abgeleitet werden. Prinzipiell können entsprechende Oligomere all dieser Sequenzen auf eine einzige Filtermembran aufgedottet werden. Demzufolge ist es möglich, ein Untersuchungsgut in einem einzigen Experiment auf das gesamte Spektrum bislang bekannter Retroviren zu testen. Im Vergleich zum Stand der Technik, demgemäß für die Identifizierung eines jeden in einer Probe vermuteten Virus ein eigener Diagosetest, insbesondere serologischer Test unter Verwendung eines speziellen Antikörpers, durchgeführt werden muß, stellt das erfindungsgemäße Verfahren somit einen bedeutenden Fortschritt dar.

Aufgrund der bekanntermaßen hohen Sensitivität der PCR und der Möglichkeit der wiederholten Reamplifikation von PCR-Produkten wird mit dem erfindungsgemäßen Verfahren zudem eine Nachweisgrenze erreicht, die von kaum einem anderen Testsystem erreicht wird.

Bei einer bevorzugten Ausführungsform des erfindungsgemäßen Verfahrens weist der "Kopf"- oder Extensionsabschnitt der erfindungsgemäßen Vorwärtsprimer und Umkehrprimer die Nukleotidsequenz GAAGGATCC auf oder besteht aus dieser. Die Nukleotidfolge GAA stellt hierbei eine sogenannte 'clamp' (=Klammer) dar und die Nukleotidfolge GGATCC repräsentiert die Schnittstelle für das Restriktionsenzym BamHI. Diese Kopf- bzw. Extensionssequenz hat sich in der Praxis sehr gut bewährt. Prinzipiell kann die Kopfsequenz aber aus jeder beliebigen Nukleotidsequenz bestehen, vorausgesetzt daß diese die Primer-Annealing-Kinetik nicht negativ beeinflußt.

Die Nukleotidsequenzen der synthetischen Oligonukleotide der RDBH-Sonden sind vorzugsweise so gewählt, daß sie zu der retroviralen Nukleinsäureregion des Reverse-Transkriptase-Gens zwischen den hoch konservierten Motiven V L P Q G und Y M/V D D I/V/L L oder zu einem Abschnitt dieser Region korrespondieren, d.h. damit übereinstimmen und/oder (im Experiment) hybridisieren können.

Da die Effizienz einer Oligonukleotid-Synthese mit der Länge des zu synthetisierenden Oligonukleotids abnimmt, ist eine Variante des erfindungsgemäßen Verfahrens vorgesehen, bei der als immobilisierte RDBH-Sonden jeweils (d.h. je Sonden bzw. je Dot) eine Mischung aus äquimolaren Mengen zweier im Vergleich kurzkettiger synthetischer Oligonukleotide eingesetzt werden. Diese korrespondieren. hintereinandergereiht zu einem längeren, vorzugsweise etwa 90 Basenpaare (bp) langen Abschnitt der Nukleinsäureregion des Reverse-Transkriptase-Gens zwischen den hoch konservierten Motiven V L P Q G und Y MN D D I/V/L L.
In einer Ausführungsform, die sich in der Praxis sehr gut bewährt hat, sind diese beiden kurzkettigen Oligonukleotide annähernd. gleich lang, und umfassen vorzugsweise ca. 45 Basenpaare.

Die Erfindung wird im folgenden anhand von Ausführungsbeispielen und dazugehörigen Figuren und Tabellen näher erläutert.

Die verwendeten Abkürzungen bedeuten:
BaEV = Baboon endogenes Retrovirus des Pavians;
ERV = endogenes Retrovirus,
ERV9 = endogenes Retrovirus Typ 9.
GaLV = Gibbonaffen Leukämie Virus;
HERV = humanes endogenes Retrovirus;
HIV = humanes Immundefizienz Virus;
HML = humane Maus Mammatumorvirus-ähnliche Sequenz;
HPLC = High Performance Liquid Chromatography
HRV5 = humanes (exogenes) Retrovirus Typ 5;
HTLV-1 = humanes adultes T-Zellen-Leukämie-Virus Typ 1;
LINE = langes, diperses (verstreut liegendes) DNA-Sequenzelement;
MMTV = Mause Mammatumor-Virus;
MoMuLV =Moloney Mäuse Leukämie Virus;
MOP= Vorwärtsprimer (forward primer) und Umkehrprimer (reversed primer) umfassende Primermischung aus degenerierten Oligonukleotiden
MPMV = Mason Pfizer Affen Virus;
PCR = Polymerase-Ketten-Reaktion
PERV = endogenes Schweine-Retrovirus (porcine endogenous retrovirus);
PBMNC = periphere mononukleäre Blutzellen (peripheral blood mononuclear cells);
RDBH = Reverse Dot Blot Hybridisierung
RT =Reverse Transkriptase

Es zeigen:
- Tabelle 1:: erfindungsgemäße Retrovirus-Typ-ABD- und -Typ-C-spezifische Primermischungen MOP-ABD und MOP-C, die jeweils Vorwärtsprimer (forward primer) und Umkehrprimer (reverse primer) umfassen und degenerierte Oligonukleotide darstellen. Zur Bescheibung der degenerierten Oligonukleotidsequenzen ist der standardisierte Einzelbuchstabenabkürzungscode der IUPAC Nomenklatur verwendet worden (siehe *European Journal of Biochemistry* 150: 15,1985). Sowohl die Vorwärtsprimer (forward primer) als auch die Umkehrprimer (reverse primer) sind, gemäß der IUPAC Konventionen und bezogen auf den DNA-Strang, in 5'-3' Richtung dargestellt. Der Grad der Degeneration, das heißt mit anderen Worten die Anzahl der verschiedenen, bei der Synthese erhältlichen möglichen konkreten Ausführungsformen dieser Primer, ist jeweils in Form der theoretisch errechneten Anzahl verschiedener Oligonukleotide angegeben.
- Tabelle 2:: Immobilisierte synthetische retrovirusspezifische Oligonukleotid-Sonden zur Herstellung von Dot-Blot Membranen. Für jeden Fleck bzw. Dot wurde eine Mischung aus äquimolaren Mengen der beiden Partner eines Oligonukleotidpaares, das zu einem 90 bp langen Abschnitt einer retrovirusspezifischen Reversen Transkriptase korrespondiert, hergestellt. Jeweils 100 Picomole dieser Mischungen wurden in der dem abgebildeten Code entsprechenden Anordnung auf die Membran aufgetragen. Zur internen Standardisierung der Hybridisierung und Autoradiographie wurde eine Verdünnungsreihe menschlicher genomischer DNA (8E - 8H) und von Oligonukleotid-Primer-Mischungen (8I - 8L) auf die Filter aufgebracht. Für jede auf dem Filter eingesetzte Oligonkleotidsequenz ist, soweit verfügbar, die Genbank- Zugriffsnummer und der Erstautor angegeben.
- Tabelle 3:: Klassifizierung von retrovirusspezifischen Oligonukleotid-Dot-Blot-Sonden: Von 61 repräsentativen Mitgliedern aller bekannten humanen exogenen und endogenen Retroviren wurde die Nukleotidsequenz des jeweiligen Reverse Transkriptase Gens in dem Bereich zwischen den hoch konservierten Domainen V L P Q G und Y M/V D D I/V/L L für die Synthese von Dot-Blot-Sonden herangezogen (Shih et al. 1989, Donehower et al. 1990). In dem hier dargestellten Experiment wurden 21 retrovirale Nukleotidsequenzen vom Typ ABD (HERV-K Superfamilie), 19 retrovirale Nukleotidsequenzen vom Typ C, 1 retrovirale Nukleotidsequenz vom Typ D und 7 Nukleotidsequenzen mit Verwandtschaft zum humanen Foamy Virus eingesetzt. Ferner wurde eine humane LINE-1 Sequenz (3L) und 6 exogene humane Retroviren (6E - 6J), sowie fünf Sonden, die zu je einem tierischen Säuger-C-Typ-Retrovirus korrespondieren und eine Sonde, die zu einem tierischen Säuger-B-Typ-Retrovirus korrespondiert (7E - 7J), getestet.
Sterne markieren mit HERV Transkripten verwandte Nukleotidsequenzen, die in Patienten mit Multipler Sklerose und Patienten mit systemischer Lupus-Erythematose gefunden wurden.
- FIG. 1.: Lokalisierung von konservierten Aminosäureabschnitten in der aminoterminalen Genregion der Reversen Transkriptase von Retroviren und Retrotransposons. Die Homologie Kernregionen V L P Q G und Y M/V D D I/V/L L wurden für die Ableitung und Herstellung der degenerierten Oligonukleotide der erfindungsgemäßen Primermischungen (MOP-ABD bzw. MOP-C) eingesetzt.
- FIG.2.: Schematische Darstellung des erfindungsgemäßen RT-PCR/RDBH Verfahrens.
- FIG. 3.: HERV Expressionsmuster in humanen PBMNCs eines gesunden Blutspenders.
Die Durchführung der Reversen Dot-Blot-Hybridisierung (RDBH) erfolgte unter Standardbedingungen mit DNA-Fragmenten, die mit den erfindungsgemäßen Primermischungen aus degenerierten Oligonukleotiden, nämlich MOP-ABD (Tabelle A) bzw. MOP-C (Tabelle B) bzw. der Kombination MOP-ABDIMOP-C (Tabelle C) amplifiziert worden waren.
- FIG.4.: HERV Expression in humanen PBMNCs nach Zugabe eines klonierten DNA-Fragments, das ein PERV RT-Gen enthält. Weniger als 10 Kopien einer endogenen Schweineretrovirus (PERV) Typ A DNA (Patience et al. 1997) konnten unter standardisierten Versuchsbedingungen noch nachgewiesen und identifiziert werden (Tafel A, Filter Code 7F). Unter den angewendeten Stringenzbedingungen wurde keine Kreuzhybridisierung von HERVs mit schweinespezifischen Amplifikationsprodukten, die von einer Schweine-DNA-Matrize gewonnen wurden, beobachtet (Tafel B).

### Beispiel 1: RNA Präparation

Von peripheren mononukleären BEutzellen (PBMNC = peripheral blood mononuclear cells) eines gesunden Blutspenders wurde die gesamt-RNA gemäß dem Guanidin Isothiocyanate/Cäsium Chlorid (GIT/CsCl) Ultrazentrifugations-Protokoll von Sambrook et al. (1989) extrahiert und in mit Diethylpyrocarbonat (DEPC)-behandeltem destillierten Wasser gelöst. Anschließend wurde die mRNA mit herkömmlichen Methoden angereichert, z.B. unter Verwendung des kommerziell erhältlichen Anreicherungskits 'Dynabeads^{TM} paramagnetic Partikel' nach den Anweisungen des Herstellers (Dynal, Hamburg, Germany). Die Nukleinsäure-Konzentration wurde mittels Spektrophotometrie bei 260 nm bestimmt. Zur Überprüfung auf etwaige Kontaminationen mit genomischer DNA wurden 50 ng von jeder mRNA-Präparation direkt, d.h. ohne zunächst einer reversen Transkription unterworfen worden zu sein, in einer Polymerase-Ketten-Reaktion (PCR) unter Verwendung der erfindungsgemäßen Primermischungen aus degenerierten Oligonucleotiden (MOP) eingesetzt. Nur solche Präparationen, die keine DNA-Spuren aufwiesen, wurden für die eigentliche PCR verwendet. Diejenigen Präparationsansätze, bei denen eine DNA-Kontamination nachweisbar war, wurden so lange mit 100 Einheiten/µg RNase-freier DNase (Roche Diagnostics, Mannheim, Germany) in 100 mM Natrium-Azetat pH 5.0, 5 mM MgSO₄ behandelt, bis die Kontroll-PCR ein negatives Ergebnis lieferte.

### Beispiel 2: Herstellung von erfindungsgemäßen Primermischungen MOP-ABD und MOP-C für die PCR.

In Tabelle 1 sind bevorzugte MOP-ABD und MOP-C-Primermischungen unter Verwendung der in Fachkreisen bekannten und geläufigen IUPAC-Nomenklatur dargestellt. Jede der Primermischungen enthält eine Mehrzahl verschiedener Vorwärtsprimer (forward primer) und Umkehrprimer (reverse primer): Die Vorwärtsprimer (forward primer) der Primermischung MOP-ABD weisen die allgemeine Nukleotidsequenz GAAGGATCCARAGTNYTDYCHCMRGGH (= **Nukleotidsequenz gemäß SEQ ID NO. 1 und ein "Kopf" am 5'-Ende**) auf, die 3456 Degenerationen, d.h. 3456 verschiedene konkrete Nukleotidsequenzen umfaßt. Die Umkehrprimer (reverse primer) der Primermischung MOP-ABD weisen die Nukleotidsequenz GAAGGATCCNWDDMKDTYATCMAYRWA (= **Nukleotidsequenz gemäß SEQ ID NO. 2 und ein "Kopf" am 5'-Ende**) auf, die 27648 Degenerationen, d.h. 27648 verschiedene konkrete Nukleotidsequenzen umfaßt. Die Vorwärtsprimer (forward primer) der Primermischung MOP-C sind durch die allgemeine Nukleotidsequenz GAAGGATCCTKKAMMSKVYTRCYHCARGGG (= **Nukleotidsequenz gemäß SEQ ID NO.3 und ein "Kopf" am 5'-Ende**) gekennzeichnet, die 3072 Degenerationen, d.h. 3072 verschiedene konkrete Nukleotidsequenzen umfaßt, und die Umkehrprimer (reverse primer) der Primermischung MOP-C weisen die Nukleotidsequenz **GAAGGATCCMDVHDRBMDKYMAYVYAHKKA (= Nukleotidsequenz gemäß SEQ ID NO. 4 und ein "Kopf" am 5'-Ende)** auf, die 8192 Degenerationen, d.h. 8192 verschiedene konkrete Nukleotidsequenzen umfaßt.
Diese Primer-Nukleotidsequenzen korrespondieren zu den hoch konservierten Homologie Kernregionen V L P Q G und Y M/V D D I/V/L L innerhalb des Reverse Transkriptase (RT) Gens aller bekannten humanen endogenen und exogenen Retroviren (siehe Fig. 1 und die Publikationen von Xiong und Eickbush 1990, Shih et al. 1989 und Donehower et al. 1990). Der als "Kopf" bezeichnete Anfang der Primernukleotidsequenzen an dem 5' Ende der jeweiligen retrovirusspezifischen Core-Homologie-Region, nämlich. die Nukleotidfolge GAAGGATCC, ist eine Extensionssequenz, die aus der sogenannten "clamp"-Sequenz GAA und der *BamHI* Restriktionsstelle GGATCC besteht.
Anstelle der hier beschriebenen "clamp"-Sequenz und der hier beschriebenen *BamHI* Restriktionsstelle kann auch eine andere "clamp"-Sequenz und/oder eine andere Schnittstelle für ein Restriktionsenzym zur Erzeugung der "Kopf" bzw. Extensionsequenz der jeweiligen Primer verwendet werden. Gundsätzlich gilt allerdings, daß die Länge dieser "Kopf" bzw. Extensionsequenz nicht mehr als die Hälfte der Primergesamtlänge betragen sollte.

Die Primermischung MOP-ABD erlaubt die separate Amplifikation der Retroviren vom Typ A, B und D, und die Primermischung MOP-C erlaubt die separate Amplifikation der Retroviren vom Typ C. Beide Primermischungen können ohne weiteres kombiniert werden und ermöglichen damit die Amplifikation aller Retrovirustypen (Typ A, B, C, und D).

### Beispiel 3: Herstellung der Sonden für die Reverse Dot Blot Hybridisierung (RDBH)

Aminosäurensequenz-Vergleiche haben gezeigt, daß die für die Reverse Transkriptase kodierenden Gene aller Retroviren und der meisten Retroelemente hoch konservierte Homologie Kernregionen aufweisen (Poch et al. 1989, Shih et al. 1989, McClure 1993, Donehower et al. 1990, Xiong and Eickbush 1990). Zwei der meist konservierten Aminosäuresequenzabschnitte sind die Aminosäuremotive V L P Q G und Y V/M D D I/V/L L (Fig. 1). Der Sequenzbereich zwischen diesen Motiven umfaßt etwa 90 Basenpaare ( d.h. ist etwa 90 bp lang) und weist eine deutlich geringere Homologie innerhalb der verschiedenen Retrovirus-Familien auf Dieser Bereich wurde zur Herstellung von retrovirusspezifischen Sonden für die RDBH herangezogen.
Dabei wurde wie folgt vorgegangen: zunächst wurden allgemein zugängliche Nukleotidsequenz-Datenbanken nach Nukleotidsequenz durchsucht, die mit der Nukleotidsequenz der Reversen Transkriptase (RT) verwandt sind. Sequenzen von exogenen und endogenen Retroviren wurden gemäß der geltenden Nomenklatur klassifiziert und hinsichtlich ihrer RT-Homologie weiter in Unterklassen untergliedert (Daten hier nicht dargestellt). Einige bisher unpublizierte HERV-Sequenzdaten wurden freundlicherweise von Martin Herrmann (1998) zur Verfügung gestellt, einige wurden selbst charakterisiert. Von allen bekannten Retrovirus-Familien wurden repräsentative Mitglieder ausgewählt (Tab. 3) und aus deren jeweiligem RT-Gen, jeweils im Bereich zwischen den hoch konservierten RT-Motiven V L P Q G und Y M/V D D I/V/L L, jeweils ein ca. 90 bp langes Fragment isoliert und als Vorlage zur Synthese entsprechender RDBH-Sonden verwendet. Da die Effizienz einer Oligonukleotid-Synthese mit der Länge des zu synthstisierenden Oligonukleotids abnimmt, wurden jeweils anstelle eines 90 pb langen Oligonukleotids (90-mer) zwei 45 bp lange Oligonukleotide (45-mers) synthetisiert und als Paar eingesetzt. Jeder Dot (Fleck) des auf die hier beschriebene Weise hergestellten Dot-Blots entspricht einer äquimolaren Mischung aus gleichen Anteilen eines Paars von 45mers aus der Gruppe der Paare, die in Tabelle 2 aufgelistet sind.

### Beispiel 4: Herstellung von Reverse-Dot-Blot-Membranen für die RDBH

Retrovirus-spezifische Oligonukleotide, die zu einem 90 bp langen Fragment der hoch konservierten Domaine des RT-Gens korrespondieren, wurden synthetisiert und mittels HPLC gereinigt. Für jede zu testende retrovirale Nukleotidsequenz wurden äquimolare Mengen der beiden Partner eines gemäß Beispiel 3 hergestellten Paars von 45-mer Oligonucleotiden zusemmengemischt und 100 Picomole dieser Paarmischung wurden in 5× SSC (1× SSC = 0,15M NaCl plus 0,015 M Natriumcitrat) gelöst und anschließend manuell oder maschinell, mit Hilfe einer handelsüblichen Dot Blot Apparatur (beispielsweise Minifold I dot blotter SRC96D von Schleicher & Schuell, Dassel Germany), auf eine handelsübliche Filtermembran (beispielsweise eine ZET Aprobe^{TM} GT blotting Membran von BioRad, Hercules CA USA) aufgetropft. Die Filter wurden in 2x SSC äquilibriert, die Oligonucleotide wurden irreversibel immobilisiert, vorzugsweise mittels UV-Crosslinking (beispielsweise mit dem kommerziell erhältlichen UV-Strahler Stratalinker™ von Stratagene, La Jolla, CA USA), und die Filter wurden anschließend an der Luft getrocknet.
Nach erfolgter Hybridisierung der Amplifikat-DNA an die kovalent auf den Membranen gebundenen RDBH-Sonden kann gebundene Amplifikat-DNA von der Dot-Blot Membran durch alkalische Denaturierung wieder gelöst werden und bei Bedarf reamplifiziert werden, um ausreichende Mengen an doppelsträngiger DNA z.B. für eine Klonierung und nachfolgende Sequenzanalyse der betreffenden Amplifikate zu erhalten.

### Beispiel 5: Reverse Transkription und Polymerase -Ketten-Reaktion (RT-PCR)

Von jedem Versuchsansatz wurden 500 ng DNA-freie mRNA in 50 µl einer Lösung aus 20 mM Tris/HCl pH 8,4, 10 mM Dithiothreitol (DTT), 50 mM KCl, 2,5 mM MgCl₂, 0,5 mM von jedem Desoxynukleosidetriphosphat (dNTP), 10 Einheiten RNasin (Promega) 30 pmol Random Hexamere Oligonucleotides (Promega) and 20 Einheiten an MLV Reverse Transkriptase (GIBCO-BRL) bei 37 °C für 1 Stunde revers transkribiert. Anschließend wurden die Ansätze denaturiert, beispielsweise durch Hitzebehandlung bei 95°C für 5 Min., und bis zur weiteren Verwendung bei - 20 °C gelagert.
Für die erfindungsgemäßen MOP-PCRs (mit MOP-ABD und/oder MOP-C) wurde jeweils ein Zwanzigstel Volumen (1/20) der cDNA-Reaktion in 50 µl einer Lösung aus 10 mM Tris/HCl pH 8,3, 50 mM KCl, 2,5 mM MgCl₂, 0,001% Gelatine, 50 pmoles der betreffenden erfindungsgemäßen Primermischung(en) aus degenerierten Oligonucleotiden, 0,25 mM von jedem Desoxynukleosidetriphosphat, und 1,25 Einheiten Taq Polymerase (GIBCO-BRL) amplifiziert. Die Versuchsansätze wurde auf Eis zubereitet und mit 50 µl Mineralöl (Sigma) überschichtet. Die Amplifikation wurde in einem handelüblichen DNA-Thermal Zykler (beispielsweise der Firma Perkin Elmer Cetus) unter Verwendung der fachbekannten "Hot-Start"-Methode durchgeführt, wobei 30 Zyklen durchlaufen wurden, die jeweils die folgenden Parameter aufwiesen: 30 Sek. bei 94°C, 4 Min.bei 50 °C und 1 Min.bei 72°C. Zum Abschluß wurde ein Extensionsschritt bei 72°C für 7 Min. durchgeführt. Die Annealingzeit, d.h. die Zeit zur Doppelstrangbildung, betrug vier Minuten, um zu gewährleisten, daß die überwiegende Zahl der in der erfindungsgemäßen Primermischung enthaltenen (degenerierten Oligonukleotid-) Primer die zu ihnen homologen Matrizen finden. Die Extensionssequenz der erfindungsgemäßen Primer hat einen stabilisierenden Effekt auf die Primer-Matrizen-Bindungskinetik, so daß die in dem ersten PCR-Zyklus gebildeten PCR-Produkte in den folgenden Zyklen wesentlich effizienter amplifiziert werden. Damit geht der Vorteil einher, daß retrovirale Matrizen selbst dann amplifiziert werden (können), wenn der exakt dazu passende Primer nicht in der erfindungsgemäßen Primermischung vorhanden ist. Darüber hinaus ist eine schnelle Klonierung der Amplifikationsprodukte z.B. für eine Sequenzüberprüfung oder für die Charakterisierung neuer RT-verwandter Nukleotidsequenzen möglich.
Die Reaktionsbedingungen für die PCR wurden im Hinblick auf die Primermenge, die Annealingzeit (Doppelstrangbildungszeit) und die Annealingtemperatur (Doppelstrangbildungstemperatur) optimiert, um eine optimale Produktausbeute zu erzielen.
Um Produkt-Kontaminationen aus vorangegangenen PCR-Experimenten und etwaige Spuren von genomischen DNA-Kontaminationen in den verwendeten Lösungen nachzuweisen, wurde eine Kontrollreaktion durchgeführt, bei der die Matrizen weggelassen wurden. Die Amplifikationsprodukte wurden auf präparativen 2.5% TBE Agarose Gelen elektrophoretisch aufgetrennt und mit Ethydiumbromid angefärbt. Banden mit einer Größe zwischen 100 und 150 bp, die mit den amplifizierten retroviralen RT-Nukleotidsequenzen korrespondierten, wurden aus dem Gel ausgeschnitten und unter Verwendung eines handelsüblichen Reinigungssets (beispielsweise des GENECLEAN II kits von BIO 101 Inc., Vista CA USA) aufgereinigt. Für die RDBH wurden ca. 50 ng der gereinigten Fragmente mit [α-³²P]dATP (3000 Ci/mmol) markiert. Die Markierung wurde mittels des Megaprime DNA labelling kit (Amersham Pharmacia Biotech, England) durchgeführt, sie kann aber selbstverständlich ebensogut auch mit Hilfe anderer gebräuchlicher Markierungsverfahren erfolgen.

### Beispiel 6: Reverse Dot Blot Hybridisierung (RDBH)

Sowohl für den Nachweis als auch für die Identifikation der amplifizierten Produkte wurde die Methode der RDBH angewendet. Diese RDBH-Methode ermöglicht eine strikte Diskriminierung (Unterscheidung) von PCR-Produkten, so daß gegebenenfalls vorhandene falsch amplifizierte Nukleotidsequenzen, die keine Verwandtschaft zu Nukleotidsequenzen von retroviralen RT-Genen aufweisen, ohne Bedeutung sind. Die hohe Stringenz der RDBH wird durch den Einsatz der erfindungsgemäßen synthetischen HERV-spezifischen Oligonukleotide erreicht, die als RDBH-Sonden auf die Dot Blot Filtermembran aufgebracht sind. Der erfindungswesentliche Vorteil dieser RDBH-Sonden- Oligonukleotide besteht darin, daß sie keine der Nukleotidsequenzen enthalten, die die degenerierten Oligonukleotide der erfindungsgemäßen PCR-Primermischungen MOP-ABD und MOP-C aufweisen (vgl. z.B. Tab. 1) und sich dadurch grundsätzlich von diesen PCR-Primer-Oligonukleotiden unterscheiden. Dieser grundsätzliche Unterschied zwischen den RDBH-Sonden-Oligonukleotiden und den PCR-Primer-Oligonukleotiden gewährleistet, daß eine Hybridisierung zwischen einer RDBH-Sonde und einem PCR-Amplifikat nur dann stattfindet, wenn die zwischen den beiden Primern befindliche Nukleotidsequenz mit dem betreffenden RDBH-Sonden-Oligonukleotid korrespondiert, d.h. wenn diese Nukleotidsequenz mit dem betreffenden RT-Nukleotidsequenzabschnitt identisch ist oder sich nur in wenigen Nukleotiden (n=3) unterscheidet. Im Idealfall sollten die hybridisierenden DNA-Sequenzen völlig identisch sein. In der Praxis unter den gegebenen Hybridisierungsbedingungen sind Unterschiede von zwei bis drei Nukleotiden tolerierbar. Infolgedessen können unter hoch stringenten Bedingungen selbst eng verwandte retrovirale Nukleotidsequenz noch voneinander unterschieden und eindeutig identifiziert werden.

Zur Vermeidung von Kreuz-Hybridisierungen wurden die optimalen Stringenzbedingungen für die RDBH bestimmt, indem Hybridisierungstemperatur, Waschtemperatur und Salzkonzentrationen variiert wurden. Die Prähybridisierung der Reverse-D-Blot-Filters wurde in verschweißten Plastiktaschen in 0,25 M Na₂HPO₄ pH 7.2, 7% Natriumdodecylsulfate (SDS), 1 mM EDTA bei 50 °C für mindestens 3 Std. durchgeführt. Für die eigentliche Hybridisierung wurden dieselben Lösungen mit 5x10⁵ CpM der markierten PCR-Amplifikate pro ml Hybridisierungsvolumen versetzt und für 16 Std. unter den gleichen Bedingungen inkubiert. Anschließend wurden die Membranen zweimal in 40 mM Na₂HFO₄, pH 7,2, 5% SDS, 1 mM EDTA und zweimal in 40 mM Na₂HPO₄, pH 7,2, 1% SDS, 1 mM EDTA, (jeweils ca 30 Min.) gewaschen. Die Untersuchung und Auswertung der Reaktion erfolgte mittels Autoradiographie.

### Beispiel 7: Analyse des HERV-Transkriptionsmusters in humanen PBMNCs mittels des erfindungsgemäßen PCR/RDBH-Verfahrens

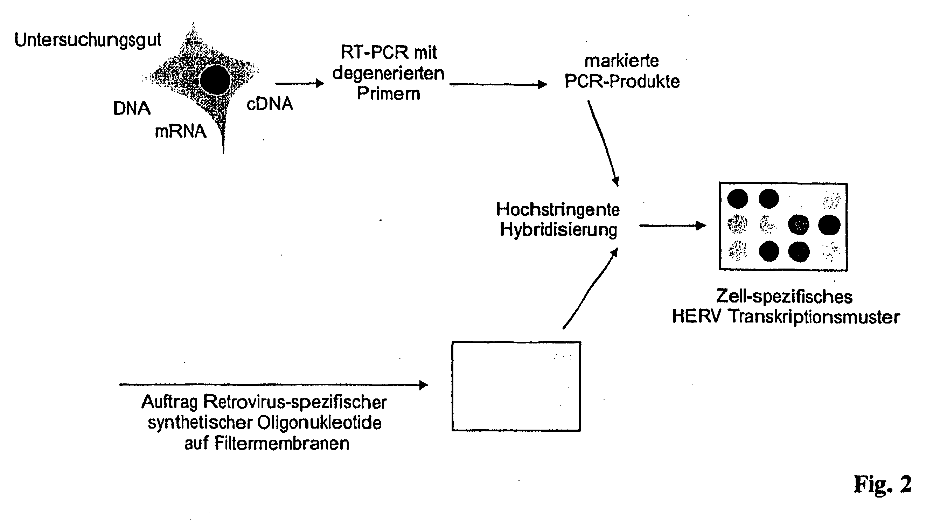

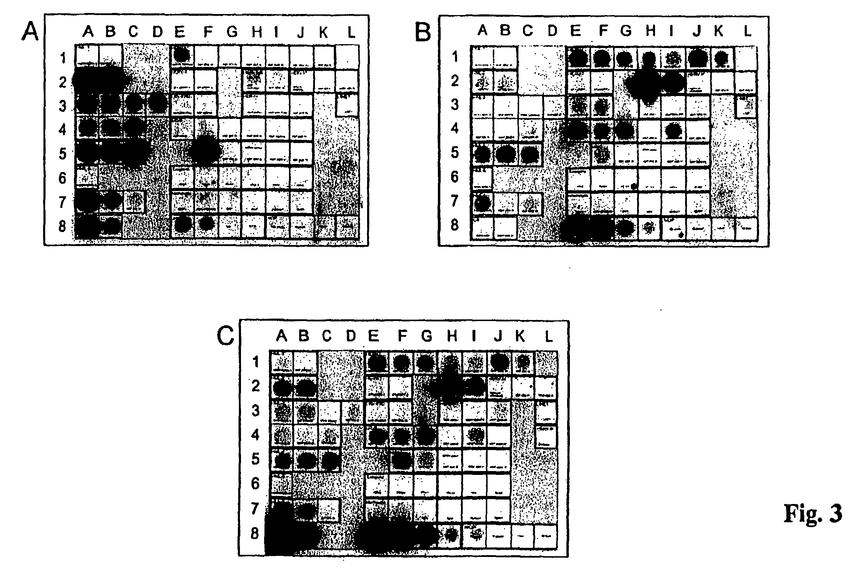

Gemäß dem in Fig. 2 dargestellten Verfahrensschema wurde zunächst aus humanen PBMNCs die Gesamt-RNA wie in Beispiel 1 beschrieben mittel gängiger Isolationstechniken extrahiert. Diese Gesamt-RNA wurde zunächst einer RT-PCR/RDBH unter Verwendung der erfindungsgemäßen Primermischung MOP-ABD unterworfen. Dabei wurden nahezu ausschließlichTyp B-verwandte HERVs, d.h. Mitglieder der HERV-K Überfamilie, nachgewiesen (Fig. 3A). Die meisten Transkripte stammten von Mitgliedern der HERV-K -Untergruppen HML-2, -3, -4, und -6. Darüber hinaus wurden Signale von HERV-KC4 verwandten Elementen (8A, 8B) und von einer weiteren HERV-K verwandten Nukleotidsequenz, die keiner der HML-Untergruppen zugeordnet werden konnte (5F), gefunden. Das beobachtete Expressionsmuster steht in Übereinstimmung mit bereits publizierten Studien, die im Ergebnis eine differenzierte Expression von HML-Elementen in humanen Geweben feststellten (Medstrand et al. 1993, Andersson et al. 1996). Außerdem wurden geringe Mengen des mit dem humanen Foamy Virus verwandten Elements HERV-L gefunden, womit die hohe Spezifität der MOP-ABD Primermischung für Typ- ABD-verwandte Elemente gezeigt ist.

Parallel zu diesem Versuch wurde die Gesamt-RNA von PBMNCs einer RT-PCR/RDBH unter Verwendung der erfindungsgemäßen Primermischung MOP-C unterworfen. Im Unterschied zur MOB-ABD-Primermischung ist die MOP-C-Primermischung nicht nur zum Priming von Typ- C-verwandten Nukleotidsequenz geeignet, sondern amplifiziert auch HERV-K-verwandte Elemente der HML-2, HML-4, und HML-6 Untergruppen. Es war eine starke Expression von HERV-E4-I-verwandten Elementen (2H and 21), von humanem Foamy Virus verwandten HERV-L Elementen (1E bis 1K) und von ERV9-verwandten HERVs (4E bis 4G, und 41) nachweisbar. Obwohl in allen RDBH-Reaktionen die gleichen Mengen an radioaktiv markierten PCR-Amplifikaten eingesetzt wurden, ergaben die auf den Membranen vorhandenen genomischen DNA-Sonden (8E bis 8H) nach der Hybridisierung mit MOP-C erzeugten PCR-Amplifikaten wesentlich stärkere Signale als nach der Hybridisierung mit MOP-ABD erzeugten PCR-Amplifikaten. Diese Befunde weisen darauf hin, daß das humane Genom signifikant mehr Kopien von Typ C-verwandten HERV-Elementen als von Typ-B-verwandten HERV Elementen enthält.

Zum Nachweis aller retroviralen Nukleotidsequenzen in einem einzigen Experiment wurden die MOP-ABD- und die MOP-C-Primermischungen in Kombination aus äquimolaren Mengen in einem erfindungsgemäßen PCR/RDBH-Verfahren eingesetzt. Dieses Experiment resultierte in einer überwiegenden Amplifikation von Typ C-verwandten Nukleotidsequenzen, während die ABD-Typ-Sequenzen unterrepräsentiert blieben (Daten hier nicht dargestellt). Aus diesem Grund wurden separate PCR Verfahren mit MOP-ABD-Primermischungen einerseits und MOP-C-Primermischungen andererseits durchgeführt und die gereinigten Amplifikationsprodukte beider Verfahren in äquimolaren Mengen kombiniert. Mit dieser Amplinkationsproduktkombination wurde dann die RDBH durchgeführt. Dabei wurde das in Fig. 3C dargestellte Signalmuster erhalten, das der theoretischen Kombination der Signalmuster der RDBH-Verfahren mit MOP-ABD- Amplinkaten gemäß Fig. 3A einerseits und mit MOP-C-Amplifikaten gemäß Fig. 3B andererseits entspricht. Dieser Befund zeigt, daß das erfindungsgemäße PCR/RDBH-Verfahren insbesondere als qualitatives Nachweisverfahren überragend gut geeignet ist.

### Beispiel 8: Nachweis der Sensitivität des erfindungsgemäßen PCR/RDBH-Verfahrens

Zur Überprüfung der Sensititvität des erfindungsgemäßen PCR/RDBH-Verfahrens im Hinblick auf einen angestrebten praktischen Einsatz in der Routinediagnostik, z.B. zum Nachweis bzw. Ausschluß einer etwaigen Interspezies-Transmission von PERV mit Xenotransplantaten, wurden Verdünnungsreihenexperimente unter Verwendung von cDNA aus humanen PBMNCs und abnehmenden Konzentrationen eines klonierten DNA-Fragments, das eine PERV RT -kodierende Region enthält (Takeuchi et al. 1998), durchgeführt. Unter standardisierten Versuchsbedingungen war in der cDNA, die aus 25 ng humaner PBMNC mRNA gewonnenen worden war, selbst noch die so geringe Menge von 10 Kopien PERV DNA nachweisbar (siehe FIG. 4A, Filter Code 7F).

Irgendwelche Kreuzhybridisierungen zwischen humanspezifischen Amplifikaten und PERV-spezifischen RDBH-Sonden wurden nicht beobachten (vgl. Fig. 3C, Filter Code 7F). Auch bei der Durchführung des PCR/RDBH-Verfahrens mit reiner Schweine-DNA als PCR Matrize (FIG. 4B) waren keine Kreuzhybridisierungen zwischen den Schweine-Amplifikaten und den humanen endogenen oder exogenen retroviralen Nukleotidsequenzen nachweisbar. Diese Ergebnisse sind ein starkes Indiz für die sehr hohe Interspezies-Spezifität des erfindungsgemäßen PCR/RDBH-Verfahrens.
Die in dem hier dargestelltenen Versuch gewonnenen Ergebnisse, nämlich das mit der murinen Typ-C Retrovirus-spezifischen Sonde (7I) erhaltene schwache Signal, weisen überraschenderweise darauf hin, daß in der Schweine-Genom-DNA ebenfalls PERVs mit einer Homology zu MoMuLV enthalten sind. Weitere Ergebnisse dieses Versuchs, nämlich die Beobachtung eines schwachen Signals mit humanen DNA-Sonden (8E, 8F) weisen ebenfalls überraschend darauf hin, daß auch das humane Genom möglicherweise PERV verwandte Nukleotidsequenzen enthält, die kein Pendant auf der eingesetzten Dot Blot Membran haben und infolgedessen vermutlich bisher noch uncharakterisiert sind. Dieser Befunde verdeutlichen den außerordentlichen Vorteil des erfindungsgemäßen PCR/RDBH-Verfahrens, nämlich die Ermöglichung des Auffindens, der Isolierung und Klonierung von bisher unbekannten DNA -Fragmenten.

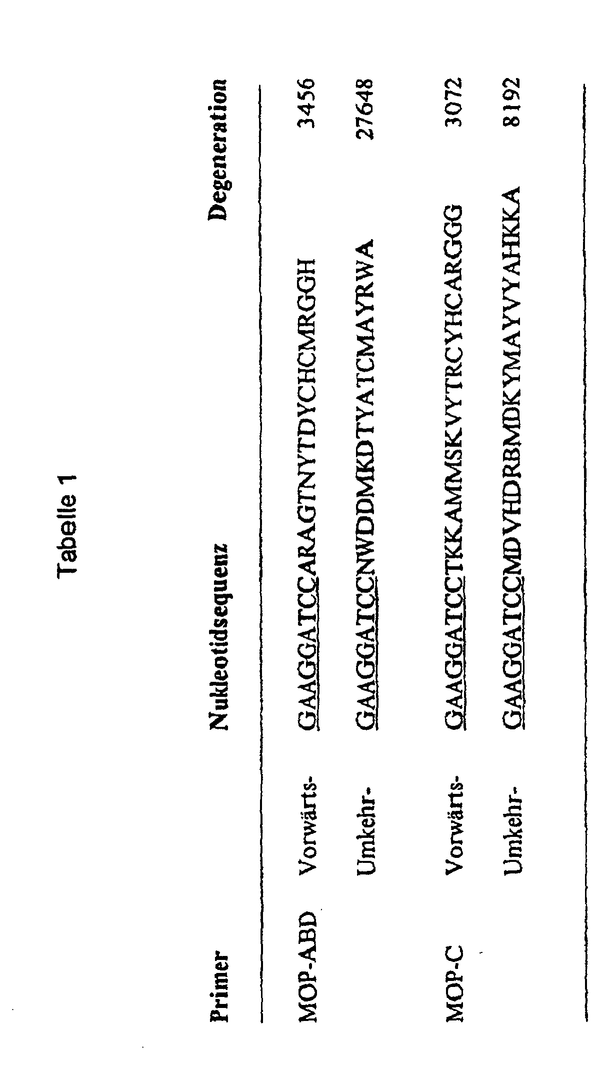

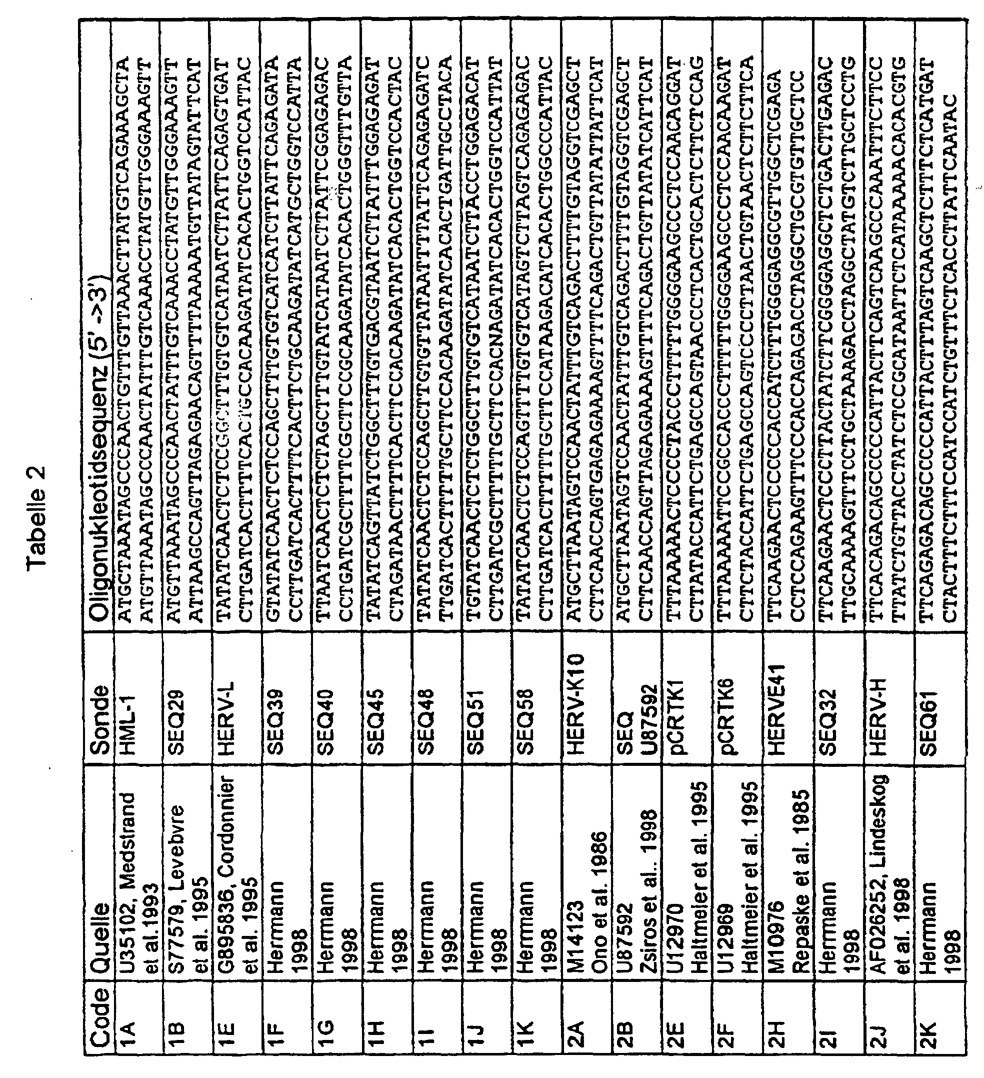

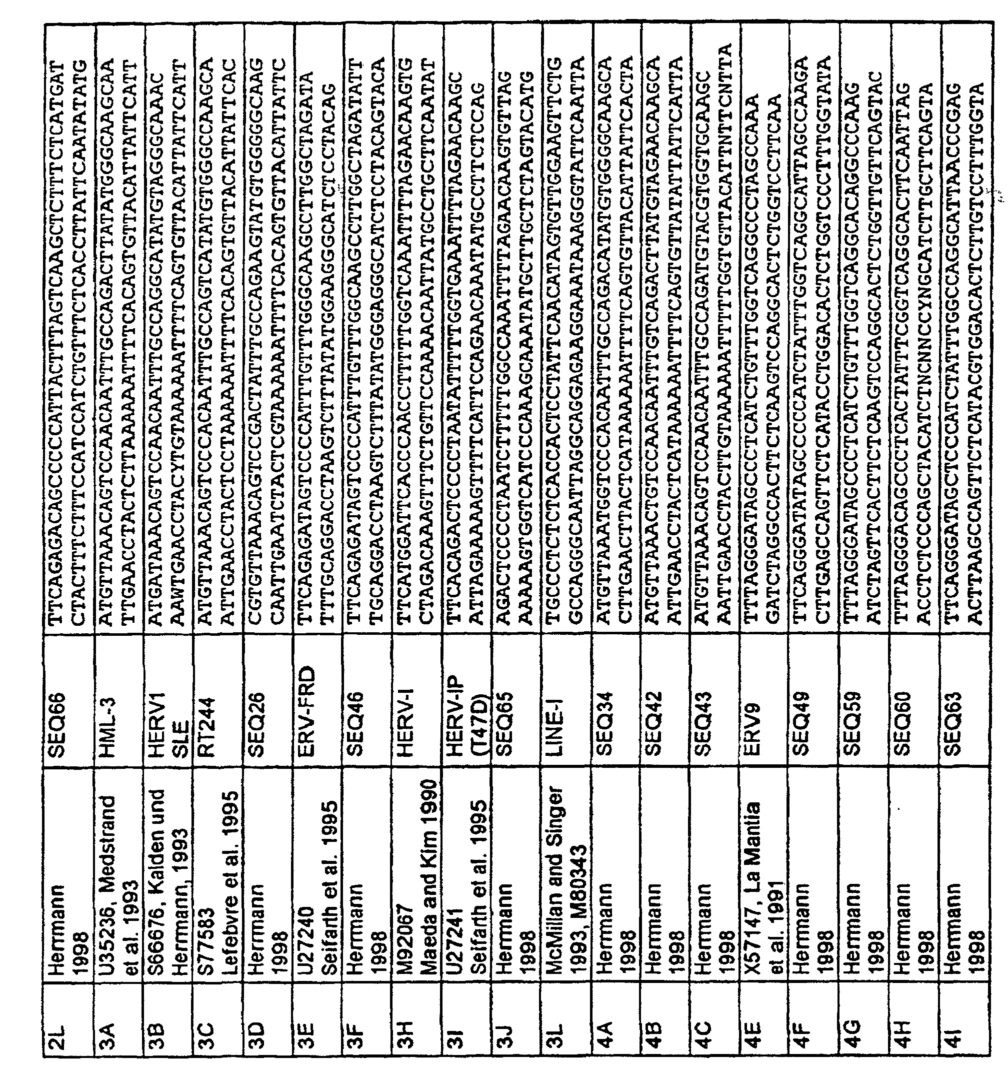

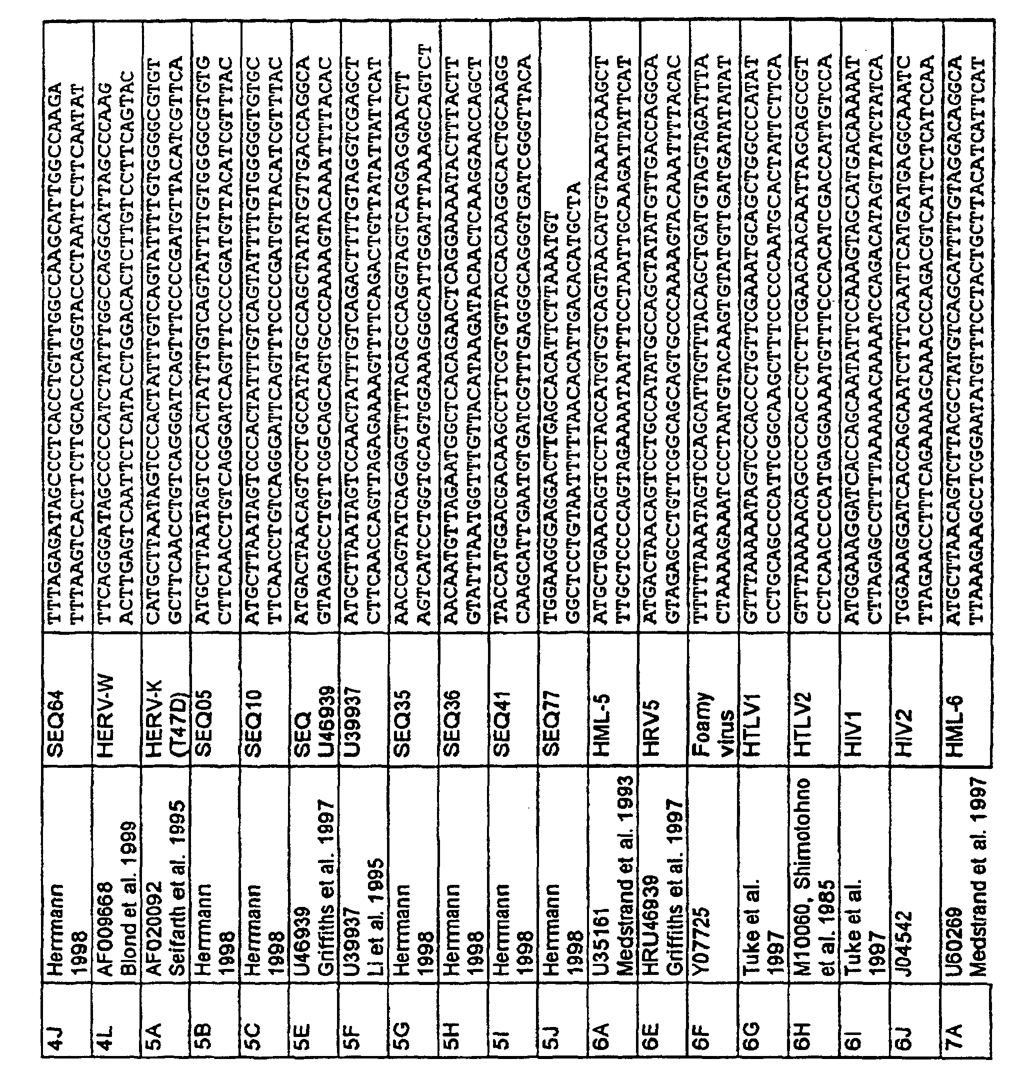

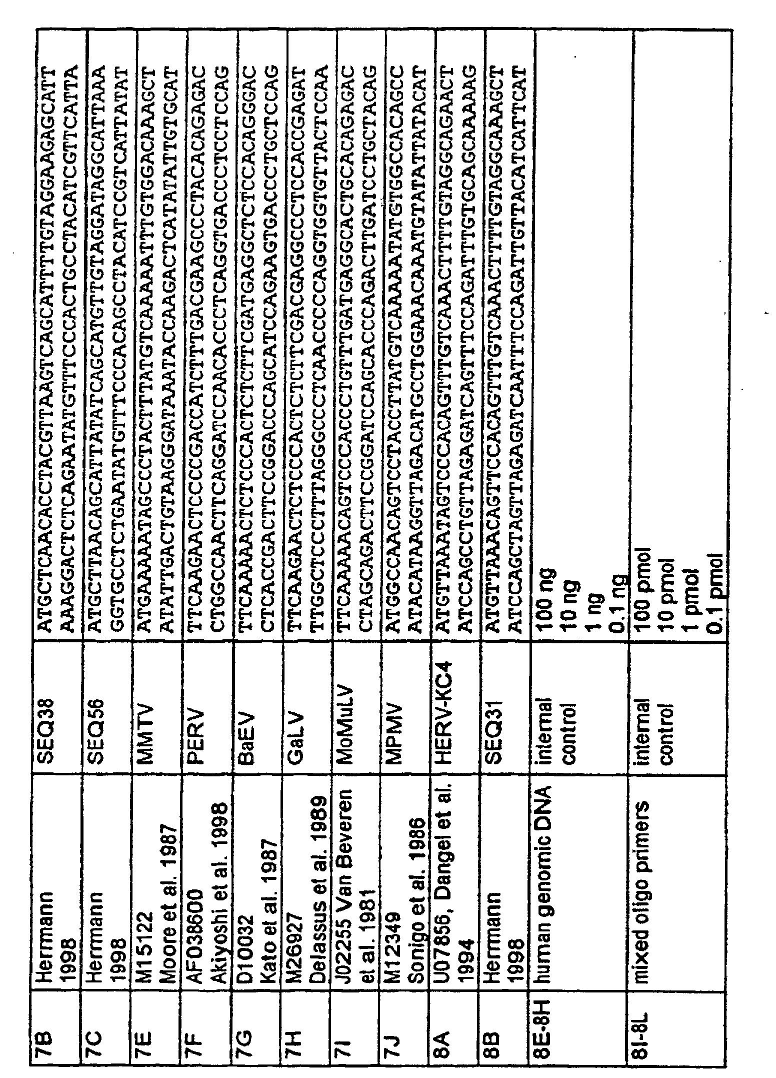

**Tabelle 3.**

| A. Humane endogene retrovirale Sequenzen | | |
|---|---|---|
| Typ B Retroviren (HERV-K-Überfamilie) | HML-1 Untergruppe | HML1 (1A) Seq29 (1B^{*}) |
| | HML-2 Untergruppe | HERV-K10 (2A) U87592 (2B) |
| | HML-3 Untergruppe | HML-3 (3A) S66676 (3B*) RT244 (3C*) Seq26 (3D*) Seq34 (4A*) Seq42 (4B*) Seq43 (4C*) |
| | HML-4 Untergruppe | HERV-K-T47D (5A) Seq05 (5B) Seq10 (5C) |
| | HML-5 Untergruppe | HML-5 (6A) |
| | HML-6 Untergruppe | HML-6 (7A) Seq38 (7B) Seq56 (7C) |
| | KC4 Untergruppe | HERV-K-C4 (8A) Seq31 (8B) |
| | | |
| | unbestimmt | U39937 (5F) |
| Typ C Retroviren | HERV-H & verwandte | AF026252 (2J) Seq61 (2K) Seq66 (2L) |
| | ERV9 & verwandte | ERV9 (4E) Seq49 (4F) Seq59 (4G) Seq60 (4H) Seq63 (4I) Seq64 (4J) |
| | ERV-FRD | ERV-FRD (3E) Seq46 (3F) |
| | HERV-ERI Familie | HERV-E(4-1) (2H) Seq32 (2I) |
| | HERV-I & verwandte | HERV-I (3H) HERV-IP-T47D (3I) Seq65 (3J) |
| | HERV-T | S71 pCRTK1 (2E) S71 pCRTK2 (2F) |
| | HERV-W | AF009668 (4L) |
| Typ D Retroviren | MPMV verwandte | Seq36 (5H) |
| Foamy Virus verwandt | HERV-L & verwandte | G895836 (1E) |
| | | Seq39 (1F) |
| | | Seq40 (1G) |
| | | Seq45 (1H) |
| | | Seq48 (1I) |
| | | Seq51 (1J) |
| | | Seq58 (1K) |
| unbestimmte retrovirale Elemente | | U46939 (5E) |
| | | Seq35 (5G) |
| | | Seq41 (5I) |
| | | Seq77 (5J) |
| humane nonvirale Retroposons | | LINE-1 (3L) |

| B. Exogene Retroviren | | |
|---|---|---|
| Humane exogene Retroviren | | HRV5 (6E) |
| | | Foamy virus (6F) |
| | | HTLV-1 (6G) |
| | | HTLV-2 (6H) |
| | | HIV-1 (6I) |
| | | HIV-2 (6J) |
| endogene Säuger-Retroviren | | MMTV(7E) |
| | | PERV (7F) |
| | | BaEV (7G) |
| | | GaLV (7H) |
| | | MoMuLV (7I) |
| | | MPMV (7J) |

### Literaturverzeichnis

Andersson, M. L., Medstrand, P., Yin, H., and Blomberg, J. (1996). Differential expression of human endogenous retroviral sequences similar to mouse mammary tumor virus in normal peripheral blood mononuclear cells. *AIDS Res.Hum.Retroviruses* **12**, 833-840.

Conrad. B., Weissmahr, R. N., Boni, J., Arcari, R., Schupbach, J., and Mach, B. (1997). A human endogenous retroviral superantigen as candidate autoimmune gene in type I diabetes. *Cell* **90**, 303-313.

Donehower, L. A., Bohannon, R. C., Ford, R. J., and Gibbs, R. A. (1990). The use of primers from highly conserved pol regions to identify uncharacterized retroviruses by the polymerase chain reaction. *J. Virol.Methods* **28**, 33-46.

Fodor, S. P., Read, J. L., Pirrung, M. C., Stryer, L., Lu, A. T., and Solas, D. (1991). Light-directed, spatially addressable parallel chemical synthesis. *Science* **251**, 767-773.

Herrmann, M. and Kalden, J. R. (1994). PCR and reverse dot hybridization for the detection of endogenous retroviral transcripts. *J Virol Methods* **46**, 333-48.

Kalden. J. R. and Herrmann, M. (1993). Autoimmune diseases in humans, e.g. autoimmune rheumatic diseases. *Intervirology* **35**, 176-185.

McClure, M. A., Thibault, K. J., Hatalski, C. G., and Lipkin, W. 1. (1993). Sequence similarity between Borna disease virus p40 and a duplicated domain within the paramyxovirus and rhabdovirus polymerase proteins. *J.Virol.* **67**, 1746. Medstrand, P. and Blomberg, J. (1993). Characterization of novel reverse transcriptase encoding human endogenous retroviral sequences similar to type A and type B retroviruses: differential transcription in normal human tissues. *J. Virol.* **67**, 6778-6787.

Patience, C., Takeuchi, Y., and Weiss, R. A. (1997). Infection of human cells by an endogenous retrovirus of pigs [see comments]. *Nat Med* **3**, 282-6.

Poch, O., Sauvaget, 1., Delarue, M., and Tordo, N. (1989). Identification of four conserved motifs among the RNA-dependent polymerase encoding elements. *EMBO J.* **8**, 3867-3874.

Sambrook, J. F. E. M. T. (1989). Molecular Cloning: A Laboratory Manual and ed. *Cold Spring Harbor Laboratory Press.*

Sauter, M., Schommer, S., Kremmer, E., Remberger, K., Dolken, G., Lemm, 1., Buck, M., Best, B., Neumann-Haefelin, D., and Mueller-Lantzsch, N. (1995). Human endogenous retrovirus K10: expression of Gag protein and detection of antibodies in patients with seminomas. J Virol 69, 414-21,

Scolnick, E. M., Vass, W. C., Howk, R. S., and Duesberg, P. H. (1979). Defective retrovirus-like 30S RNA species of rat and mouse cells are infectious if packaged by type C helper virus. *J. Virol.* **29***,* 964-972.

Seifarth, W., Baust, C., Murr, A., Skladny, H., Krieg-Schneider, F., Blusch, J., Werner, T., Hehlmann, R., and Leib-Mosch, C. (1998). Proviral structure, chromosomal location, and expression of HERV-K- T47D, a novel human endogenous retrovirus derived from T47D particles. *J Virol* **72**, 8384-91.

Seifarth. W., Sktadny, H., Krieg-Schneider, F., Reichert, A., Hehlmann. R., and Leib-Mosch, C. (1995). Retrovirus-like particles released from the human breast cancer cell line T47-D display type B- and C-related endogenous retroviral sequences. *J Virol* **69**, 6408-16.

Sherwin, S. A., Rapp, U. R:, Benveniste, R: E., Sen, A., and Todaro, G. J. (1978). Rescue of endogenous 30S retroviral sequences from mouse cells by baboon type C virus. *J. Virol.* **26**, 257-264.

Shih, A., Misra, R., and Rush, M. G. (1989). Detection of multiple, novel reverse transcriptase coding sequences in human nucleic acids: relation to primate retroviruses. *J. Virol.* **63**, 64-75.

Takeuchi, Y., Patience, C., Magre, S., Weiss, R. A., Banerjee, P. T., Le Tissier, P., and Stoye, J. P. (1998). Host range and interference studies of three classes of pig endogenous retrovirus. *J. Virol.* **72**, 9986-9991.

Takeuchi, Y., Simpson, G., Vile, R. G., Weiss, R. A., and Collins, M. K. (1992). Retroviral pseudotypes produced by rescue of a Moloney murine leukemia virus vector by C-type, but not D-type, retroviruses. *Virology* **186**, 792-794.

Tuke, P. W., Perron, H., Bedin, F., Beseme, F., and Garson, J. A. (1997). Development of a pan-retrovirus detection system for multiple sclerosis studies. *Acta Neurol.Scand.Suppl* **169**, 16-21.

Vogetseder, W., Dumfahrt, A., Mayersbach, P., Schonitzer, D., and Dierich, M. P. (1993). Antibodies in human sera recognizing a recombinant outer membrane protein encoded by the envelope gene of the human endogenous retrovirus K. *AIDS Res Hum Retroviruses* **9**,687-94.

Xiong, Y. and Eickbush, T. H. (1990). Origin and evolution of retroelements based upon their reverse transcriptase sequences. *Embo J* **9**, 3353-62.

Organization Applicant
----------------------
Street :
City :
State :
Country :
PostalCode :
PhoneNumber :
FaxNumber :
EmailAddress :
<110> OrganizationName : Ruprecht-Karls-Universität Heidelberg

Organization Applicant
----------------------
Street : Seminarstrasse 2
City : Heidelberg
State :
Country : Deutschland
PostalCode : 69117
PhoneNumber :
FaxNumber :
EmailAddress :
<110> OrganizationName : Ruprecht-Karls-Universität Heidelberg

Application Project
-------------------
<120> Title : Verfahren zum spezifischen Nachweis und zur Identi
fizierung retroviraler Nukleinsäuren/Retroviren in einem Untersu
chungsgut
<130> AppFileReference : DE19921419.0
<140> CurrentAppNumber : 00931021.0-2402
<141> CurrentFilingDate : 2003-01-07

Sequence
--------
<213> OrganismName : Homo sapiens
<400> PreSequenceString :
aragtnytdy chcmrggh
18
<212> Type : DNA
<211> Length : 18
SequenceName : SEQID1
SequenceDescription :

Feature
-------
Sequence: SEQID1:
<221> FeatureKey : misc_feature
<222> LocationFrom : 6
<222> LocationTo : 6

Other Information : A or C or G or T
CDSJoin : No

Sequence
--------
<213> OrganismName : Homo sapiens
<400> PreSequenceString :
nwddmkdtya tcmayrwa
18
<212> Type : DNA
<211> Length : 18
SequenceName : SEQID2
SequenceDescription :

Feature
-------
Sequence: SEQID2:
<221> FeatureKey : misc_feature
<222> LocationFrom : 1
<222> LocationTo : 1
Other Information : A or C or G or T
CDSJoin : No

Sequence
--------
<213> OrganismName : Homo sapiens
<400> PreSequenceString :
tkkammskvy trcyhcargg g
21
<212> Type : DNA
<211> Length : 21
SequenceName : SEQID3
SequenceDescription :

Sequence
--------
<213> OrganismName : Homo sapiens
<400> PreSequenceString :
mdvhdrbmdk ymayvyahkk a
21
<212> Type : DNA
<211> Length : 21
SequenceName : SEQID4
SequenceDescription :

Feature
-------
Sequence: SEQID4:
<221> FeatureKey : SEQID5
<222> LocationFrom : 1

<222> LocationTo : 10
      Other Information :
      CDSJoin : No

Feature
-------
Sequence: SEQID4:
<221> FeatureKey : misc_feature
<222> LocationFrom : 1
<222> LocationTo : 10
      Other Information :
      CDSJoin : No

Sequence
--------
<213> OrganismName : artificial
<400> PreSequenceString :
gaaggatcc
        9
<212> Type : DNA
<211> Length : 9
      SequenceName : SEQID5
      SequenceDescription :

Feature
-------
Sequence: SEQID5:
<221> FeatureKey : misc_feature
<222> LocationFrom : 1
<222> LocationTo : 9
      Other Information : arbitrary head sequence
      CDSJoin : No

## Patentansprüche

1. Aus Vorwärtsprimern und Umkehrprimern bestehende Primermischung ("MOP") für die PCR, **dadurch gekennzeichnet,**
- **daß** Vorwärtsprimer und Umkehrprimer degenerierte Oligonukleotide sind,
- **daß** entweder ("MOP-ABD")
die Vorwärtsprimer eine Nukleotidesquenz aufweisen, die aus der Nukleotidsequenz gemäß SEQ ID NO. 1 mit 3456 Degenerationen und einem "Kopf" am 5'-Ende besteht, und
die Umkehrprimer eine Nukleotidesquenz aufweisen, die aus der Nukleotidsequenz gemäß SEQ ID NO. 2 mit 27648 Degenerationen und einem "Kopf" am 5'-Ende besteht,
- oder daß ("MOP-C")
die Vorwärtsprimer eine Nukleotidesquenz aufweisen, die aus der Nukleotidsequenz gemäß SEQ ID NO. 3 mit 3072 Degenerationen und einemn "Kopf" am 5'-Ende besteht, und
die Umkehrprimer eine Nukleotidsequenz aufweisen, die aus der Nukleotidsequenz gemäß SEQ ID NO. 4 mit 8192 Degenerationen und einen "Kopf" am 5'-Ende besteht,
- und **daß** "Kopf" für eine Nukleotidsequenz steht, die eine Schnittstelle für ein Restriktionsenzym und eine Klammer-Sequenz, die der Stabilisierung der Schnittstellen sequenz dient, am 5'-Ende dieser Schnittstelle umfaßt, wobei die Länge dieser Nukleotidsequenz die halbe Länge der kompletten Nukleotidsequenz des Vorwärts- oder Umkehrprimers nicht überschreitet.

2. Primermischung nach Anspruch 1, **dadurch gekennzeichnet**,
das der "Kopf"-Abschnitt der Vorwärtsprimer und Umkehrprimer die Nukleotidsequenz GAAGGATCC gemäß SEQ ID NO. 5 aufweist.

3. Verfahren zum spezifischen Nachweis und zur Identifizierung retroviraler Nukleinsäuren / Retroviren in einem Untersuchungsgut, **gekennzeichnet durch** Art und Reihenfolge der nachstehend genannten Maßnahmen:
- Isolierung der Nukleinsäuren, nämlich DNS- und/oder RNS, aus dem Untersuchungsgut,
- Durchführung einer PCR mit den isolierten DNS oder einer RT-PCR mit den isolierten RNS unter Verwendung von einer oder beiden Primermischungen gemäß Anspruch 1
- Aufreinigung der erhaltenen (RT-) PCR-Amplifikate und Einsatz derselben in einem Reverse-Dot-Blot-Hybridisierungs-Verfahren unter Verwendung immobilisierter Reverse-Dot-Blot-Hybridisierungs-Sonden, wobei diese Sonden synthetische Oligonukleotide umfassen, deren Nukleotidsequenz der retroviralen NukJeotidsequenz des retroviruspezifischen Reverse-Transkriptase-Gens der mit dem betreffenden Dot nachzuweisenden Virusart oder einem Abschnitt einer solchen retroviralen Nukleotidsequenz entspricht und keine Überlappung mit den Nukleotidsequenzen der Vorwärtsprimer und Umkehrprimer der in der PCR oder RT-PCR eingesetzten Primermischungen aufweist.

4. Verfahren nach Anspruch 3, **dadurch gekennzeichnet,**
**daß** die Nukleotidsequenzen der synthetischen Oligonukleotide der Reverse-Dot-Blot-Hybridisierungs-Sonden zu der retroviralen Nukleinsäureregion des Reverse-Transkriptase-Gens zwischen den hoch konservierten Motiven V L P Q G und Y M/V D D I/V/L L oder zu einem Abschnitt dieser Region korrespondieren.

5. Verfahren nach einem der Ansprüche 3 oder 4, **dadurch gekennzeichnet,**
**daß** als immobilisierte Reverse-Dot-Blot-Hybridisierungs-Sonden jeweils eine Mischung aus äquimolaren Mengen der beiden Partner eines Paars synthetischer Oligonukleotide, das zusammen zu einem Abschnitt aus der Nukleinsäureregion des Reverse-Transkriptase-Gens zwischen den hoch konservierten Motiven V L P Q G und Y M/V D D I/V/L L korrespondiert, eingesetzt wird.

6. Verfahren nach Anspruch 5, **dadurch gekennzeichnet,**
**daß** der Abschnitt 90 bp lang ist.

7. Verfahren nach Anspruch 5 oder 6 **dadurch gekennzeichnet,**
**daß** die beiden Partner des Paars synthetischer Oligonukleotide annähernd gleich lang sind.

8. Verwendung von einem oder mehreren synthetischen Oligonukleotid(en), dessen/deren Nukleotidsequenz(en) mit der Nukleinsäureregion eines retrovirusspizifischen Reverse-Transkriptase-Gens zwischen den hoch konservierten Motiven V L P Q G und Y M/V D D I/V/L L oder mit einem Abschnitt dieser Nukleinsäureregion korrespondiert/korrespondieren, als Reverse-Dot-Blot-Hybridisierungs-Sonde(n) in einem Verfahren nach einem der Ansprüche 3 bis 6.

9. Verwendung nach Anspruch 8, **dadurch gekennzeichnet, daß** äquimolare Mengen zweier synthetischer Oligonukleotide, die hintereinandergereiht zu einem Abschnitt aus der Nukleinsäureregion des Reverse-Transkriptase-Gens zwischen den hoch konservierten Motiven V L P Q G und Y M/V D D I/V/L L korrespondieren, eingesetzt sind.

10. Diagnosekit zum spezifischen Nachweis und zur Identifizierung retroviraler Nukleinsäuren / Retroviren in einem beliebigen Untersuchungsgut, umfassend wenigstens eine der aus Vorwärtsprimern und Umkehrprimern bestehenden Primermischungen für die PCR gemäß Anspruch 1 und wenigstens eine Reverse-Dot-Blot-Hybridisierungs-Sonde gemäß Anspruch 8 oder Anspruch 9.

## Claims

1. A primer mixture ("MOP") consisting of forward and reverse primers for PCR, **characterised in**
**that** the forward and reverse primers are degenerated oligonucleotides,
**that** either ("MOP-ABD")
the forward primers exhibit a nucleotide sequence consisting of the nucleotide sequence according to SEQ ID NO. 1 with 3456 degenerations and a "head" at the 5' end,
and the reverse primers exhibit a nucleotide sequence consisting of the nucleotide sequence according to SEQ ID NO.2 with 27648 degenerations and a "head" at the 5' end,
or ("MOP-C")
the forward primers exhibits a nucleotide sequences consisting of the nucleotide sequence according to SEQ ID NO.3 with 3072 degenerations and a "head" at the 5' end and
the reverse primers exhibit a nucleotide sequences consisting of the nucleotide sequence according to SEQ ID NO. 4 with 8192 degenerations and a "head" at the 5' end and
and **that** "head" stands for a nucleotide sequence which comprises an interface for a restriction enzyme and a clamp sequence for stabilizing the interface sequence at the 5' end of this interface, wherein the length of said nucleotide sequence does not exceed half the length of the complete nucleotide sequence of the forward or reverse primer.

2. Primer mixture according to claim 1, **characterised in that** the "head" section of the forward primers and reverse primers exhibit the nucleotide sequence GAAGGATCC according to SEQ ID NO. 5.

3. Method for the specific detection and identification of retroviral nucleic acids/retrovirus in a specimen, **characterised by** type and sequence of the measures specified hereinafter:
- Isolation of nucleic acids, namely DNA and/or RNA from the specimen,
- Carrying out a PCR with the isolated DNA or an RT-PCR with the isolated RNA using. one or both primer mixtures according to Claim 1,
- Purging the (RT)-PCR amplificates obtained and using these in an reverse dot blot hybridisation method using immobilised reverse dot blot hybridisation probes, wherein these probes comprise synthetic oligonucleotides whose nucleotide sequence corresponds to the retroviral nucleotide sequence of the retrovirus-specific reverse-transcriptase gene of the virus type to be detected with the relevant dot or a section of such a retroviral nucleotide sequence and exhibits no overlapping with the nucleotide sequences of the forward primer and the reverse primer of the primer mixtures used in the PCR or RT-PCR.

4. Method according to claim 3, **characterised in that** the nucleotide sequences of the synthetic oligonucleotides of the reverse dot blot hybridisation probes correspond to the retroviral nucleic acid region of the reverse transcriptase gene between the highly conserved motifs V L P Q G and Y M/V D D I/V/L L or to a section of this region.

5. Method according to one of claims 3 or 4, **characterised in that** a mixture of equimolar quantities of both partners of a pair of synthetic oligonucleotides, which together correspond to a section from the nucleic acid region of the reverse transcriptase gene between the highly conserved motifs V L P Q G and Y M/V D D I/V/L L , is in each case used as immobilised reverse dot blot hybridisation probes.

6. Method according to claim 5, **characterised in that** the section is 90 bp long.

7. Method according to claim 5 or 6, **characterised in that** both partners of the pair of synthetic oligonucleotides have approximately the same length.

8. Use of one or several synthetic oligonucleotide(s) whose nucleotide sequence(s) correspond(s) to the nucleic acid region of a retrovirus-specific reverse transcriptase gene between the highly conserved motifs V L P Q G and Y M/V D D I/V/L L or to a section of this nucleic acid region as reverse dot blot hybridisation probe(s) in a method according to one of Claims 3 to 6.

9. Use according to claim 8, **characterised in that** equimolar quantities of two synthetic oligonucleotides come into operation which positioned one after the other correspond to a section from the nucleic acid region of the reverse transcriptase gene between the highly conserved motifs V L P Q G and Y M/V D D I/V/L L.

10. Diagnosis kit for the specific detection and identification of retroviral nucleic acids/retroviruses in an arbitrary specimen, comprising at least one of the primer mixtures consisting of forward and reverse primers for the PCR according to Claim 1 and at least one reverse dot blot hybridisation probe according to Claim 8 or Claim 9.

## Revendications

1. Mélange d'amorces (« MOP ») constitué d'amorces avant et d'amorces inverses pour la réaction en chaîne de la polymérase (PCR), **caractérisé**
• **en ce que** les amorces avant et les amorces inverses sont des oligonucléotides dégénérés,
• **en ce que** soit (« MOP-ABD »)
les amorces avant ont une séquence de nucléotides constituée de la séquence de nucléotides selon la séquence SEQ ID n° 1 avec 3 456 dégénérations et d'une «tête» à l'extrémité 5', et
les amorces inverses ont une séquence des nucléotides constituée de la séquence de nucléotides selon la séquence SEQ ID n° 2 avec 27 648 dégénérations et une «tête» à l'extrémité 5',
• ou en ce que (« MOP-C »)
les amorces avant ont une séquence de nucléotides constituée de la séquence de nucléotides selon la séquence SEQ ID n° 3 avec 3 072 dégénérations et une «tête» à l'extrémité 5', et
les amorces inverses ont une séquence de nucléotides constituée de la séquence de nucléotides selon la séquence SEQ ID n° 4 avec 8 192 dégénérations et une « tête » à l'extrémité 5',
• et **en ce que** «tête» représente une séquence de nucléotides qui comprend une interface pour un enzyme de restriction et une séquence de blocage qui sert à stabiliser la séquence d'interface à l'extrémité 5' de cette interface, la longueur de cette séquence de nucléotides ne dépassant pas la moitié de la longueur de la séquence de nucléotides complète de l'amorce avant ou de l'amorce inverse.

2. Mélange d'amorces selon la revendication 1, **caractérisé en ce que** la section de « tête » des amorces avant et des amorces inverse présente la séquence de nucléotides GAAGGATCC selon la séquence SEQ ID n° 5.

3. Procédé de détection spécifique et d'identification d'acides nucléiques rétroviraux / de rétrovirus dans une élément à analyser, **caractérisé par** le genre et l'ordre de succession des mesures citées ci-dessous :
• isolation des acides nucléiques, à savoir ADN ou ARN, de l'élément à analyser,
• réalisation d'une PCR avec les ADN isolés ou d'une RT-PCR avec les ARN isolés en utilisant l'un ou les deux mélanges d'amorces selon la revendication 1,
• purification du produit d'amplification (RT-)PCR obtenu et utilisation de ce dernier dans un procédé d'hybridation dot blot inverse en utilisant des sondes d'hybridation dot blot inverse immobilisées, ces sondes comprenant des oligonucléotides synthétiques dont la séquence de nucléotides correspond à la séquence de nucléotides rétrovirale du gène de la transcriptase inverse spécifique au rétrovirus du type de virus à détecté avec le dot considéré, ou une partie d'une telle séquence de nucléotides rétrovirale, et qui ne présente pas de recouvrement avec les séquences de nuclëotides des amorces avant et des amorces inverses des mélanges d'amorces utilisés dans la PCR ou la RT-PCR.

4. Procédé selon la revendication 3, **caractérisé en ce que** les séquences de nucléotides des oligonucléotides synthétiques des sondes d'hybridation dot blot inverse correspondent à la région de l'acide nucléique rétrovirale du gène de la transcriptase inverse comprise entre les motifs très bien conservés V L P Q G et Y MN D D I/V/L L ou à une section d'une telle région.

5. Procédé selon la revendication 3 ou 4, **caractérisé en ce qu'**on utilise comme sondes d'hybridation dot blot inverse immobilisées un mélange de quantités équimolaires des deux partenaires d'une paire d'un oligonucléotide synthétique qui constituent ensemble une section de la région d'acide nucléique du gène de la transcriptase inverse comprise entre les motifs très bien conservés V L P Q G et Y MN D D IN/L L.

6. Procédé selon la revendication 5, **caractérisé en ce que** la section est longue de 90 paires de bases.

7. Procédé selon la revendication 5 ou 6, **caractérisé en ce que** les deux partenaires de la paire d'oligonucléotides synthétiques ont sensiblement la même longueur.

8. Utilisation comme sonde(s) dot blot inverse dans un procédé selon l'une des revendications 3 à 6 d'un ou plusieurs oligonucléotide(s) synthétique(s), dont sa/leurs séquence(s) de nucléotides correspond(ent) à la région d'acide nucléique d'un gène de la transcriptase inverse spécifique au rétrovirus comprise entre les motifs très bien conservés V L P Q G et Y MN D D I/V/L L, ou à une section de cette région d'acide nucléique.

9. Utilisation selon la revendication 8, **caractérisé en ce qu'**on utilise des quantités équimolaires de deux oligonucléotides synthétiques qui correspondent mis l'un derrière l'autre à une section de la région de l'acide nucléique du gène de la transcriptase inverse comprise entre les motifs très bien conservés V L P Q G et Y M/V D D I/V/L L.

10. Kit de diagnostic pour la détection spécifique et l'identification d'acides nucléiques rétroviraux / de rétrovirus dans n'importe quel élément à analyser, comprenant au moins un des mélanges d'amorces constitués d'amorces avant et d'amorces inverses prou la PCR selon la revendication 1 et au moins une sonde d'hybridation dot blot inverse selon la revendication 8 ou 9.
